# EUROPÄISCHE PATENTSCHRIFT

(11) **EP 1 639 066 B1**
(45) Veröffentlichungstag und Bekanntmachung des Hinweises auf die Patenterteilung: **01.12.2010**
(21) Anmeldenummer: 04729859.1
(22) Anmeldetag: 28.04.2004
(51) Int. Cl.: C11D 1/62, C11D 1/94, C11D 17/00, A61K 8/41, A61K 8/34, A61K 8/44, A61Q 5/12

(54) **FROSTRESISTENTE KONDITIONIERMITTEL**
FROST-RESISTANT CONDITIONING AGENT
AGENTS DE CONDITIONNEMENT RESISTANTS AU GEL

(30) Priorität: 08.05.2003 DE 10320433
(43) Veröffentlichungstag der Anmeldung: 29.03.2006
(73) Patentinhaber: Henkel AG & Co. KGaA, 40589 Düsseldorf (DE)
(72) Erfinder: MADLE, Petra-Stefanie, A-1090 Wien (AT); LASKE, Christian, A-2380 Perchtoldsdorf (AT); SCHEFFLER, Karl-Heinz, 40589 Düsseldorf (DE); PLAMBERGER, Johannes, A-1030 Wien (AT)
(86) Internationale Anmeldenummer: PCT/EP2004/004454
(87) Internationale Veröffentlichungsnummer: WO 2004/099354

(56) Entgegenhaltungen:
- EP-A- 0 937 771
- WO-A-94/17169
- WO-A-97/03169
- WO-A-99/09122
- US-A1- 2002 160 926
- US-B1- 6 180 594
- US-B1- 6 376 455

## Beschreibung

Die Erfindung betrifft trübe frostresistente, flüssige Konditioniermittel sowie ein Verfahren zur Herstellung solcher Mittel. Weiterhin betrifft die Erfindung die Verwendung solcher Mittel zum Konditionieren von Textilien sowie die Verwendung solcher Mittel zum Konditionieren keratiner Fasern.

Die Bereitstellung frostresistenter Konditioniermittel, beispielsweise von Textilkonditioniermitteln, die ihren Einsatz in der Wäschenachbehandlung und Textilavivage finden, ist vom Verbraucher wie vom Handel naturgemäß gerade in solchen Gegenden erwünscht, in denen die klimatischen Gegebenheiten dies erfordern. Es ist ein bekanntes Problem, daß Konditioniermittel wie z. B. Weichspülerformulierungen oftmals eine nur unbefriedigende Kältestabilität aufweisen, d. h. daß sie nach Kälteeinfluss, insbesondere nach Lagerung bei Temperaturen unterhalb 0°C nicht nur erheblich verdicken, sondern zudem Ausfällungen, irreversible Vergelungen oder Phasenseparationen usw. zeigen. Als Folge solcher Ausfällungen, Vergelungen oder Phasenseparationen, die zudem einen erheblichen ästhetischen Mangel darstellen, verlieren die Konditioniermittel in sehr großem Ausmaß an Funktionalität und können nicht mehr sachgerecht verwendet werden.

Die Problematik der mangelnden Kältestabilität solcher Konditioniermittel und entsprechende Lösungsansätze zu deren Vermeidung sind im Stand der Technik umfangreich beschrieben, besonders was das Segment der Textilweichspüler betrifft.

So beschreibt die europäische Patentanmeldung EP 0 280 550 A1**,** daß die Zugabe eines Niotensids zu einer Zusammensetzung, die eine kationische Textilweichmacherkomponente sowie eine Fettsäure enthält, zu wäßrigen Textilweichmacherzusammensetzungen führt, die selbst nach mehreren Einfrier-/Auftau-Zyklen stabil und verwendungsfähig sind.

Die europäische Patentanmeldung EP 0 728 178 A1 beschreibt vor einem ähnlichen Hintergrund ein Wäschenachbehandlungsmittel, das neben einer typischen quartären Ammoniumverbindung mit zumindest einer Esterbindung zusätzlich eine zwitterionische Ammoniumverbindung enthält.

In der europäischen Patentanmeldung EP 0 763 592 A1 werden wiederum stabilisierte Textilweichmacherzusammensetzungen beschrieben, die neben einer bioabbaubaren Textilweichmacherverbindung noch eine Fettsäureverbindung in einem bestimmten Gewichtsverhältnis beinhalten.

Solche und ähnliche Lösungsansätze sind zwar hilfreich, bleiben aber letztlich verbesserungswürdig, da sie die Wünsche der Verbraucher und des Handels nicht ausreichend erfüllen können. Unbefriedigt bleibt bei vorgenannten Lösungsansätzen beispielsweise das Bedürfnis der Kunden, die entsprechenden Konditioniermittel auch bei Temperaturen unter 0°C zu handhaben. Solche widrigen Temperaturen sind in vielen Regionen der Welt an der Tagesordnung und der dortige Verbraucher hat oftmals nicht die Möglichkeiten für beheizten Lagerraum zu sorgen. Will er dann beispielsweise ein Konditioniermittel verwenden, das er aus Platzgründen in einem nicht beheiztem Lagerraum aufbewahrt, so muß er erst abwarten, bis das Mittel wieder aufgetaut ist, bevor er es verwenden kann. Es ist in einem solchen Fall ein großer Vorteil für den Verbraucher, ein Konditioniermittel zur Hand zu haben, daß auch bei Frost nicht erstarrt.

Solche Mittel, nämlich klare oder transluzente, konzentrierte, stabile Textilweichmacherzusammensetzungen werden in der europäischen Patentanmeldung EP 0 842 250 A1 beschrieben. Diese Textilweichmacherzusammensetzungen zeichnen sich u. a. dadurch aus, daß sie neben einer guten Gefrier/Auftau-Beständigkeit auch bei Temperaturen in einem bestimmten Bereich unterhalb 0°C nicht gefrieren. Diese klaren oder transluzenten Textilweichmacherzusammensetzungen bestehen aus einer quartären Ammoniumverbindung und einem sogenannten Hauptlösungsmittel, das einen ClogP-Wert zwischen 0,15 und 0,64 aufweist und zudem einen gewissen Grad an Asymmetrie besitzt, beispielsweise 1-Isopropyl-1,2-cyclobutandiol, wobei dieses Hauptlösungsmittel eine unzureichende Menge bestimmter anderer Lösungsmittel enthalten muß, beispielsweise 2,2,4-Trimethyl-1,3-pentandiol.

Weitere wässrige Textilweichmacherzusammensetzungen mit Lösungsmitteln werden in US 2002/0160926 A1 sowie WO-A-9417169 beschrieben.

Die Bereitstellung entsprechender trüber Konditioniermittel mit ähnlichem Eigenschaftsprofil wird in der EP 0 842 250 A1 jedoch nicht beschrieben, die dortige Offenbarung führt zwangsläufig zu klaren oder transluzenten Textilweichmacherzusammensetzungen.

Demgegenüber steht der Wunsch und das Bedürfnis der Kunden und damit des Handels nach trüben Konditioniermitteln mit ähnlichem Eigenschaftsprofil. Zum einen erbringen trübe Konditioniermittel eine bessere Weichheitsleistung, was auf die betreffenden quartären Ammoniumverbindungen bzw. die zu Grunde liegenden Fettsäuren zurückzuführen ist. Darüber hinaus basieren die trüben Konditioniermittel bzw. die darin enthaltenen quartären Ammoniumverbindungen auf billigeren Rohstoffen. Beispielsweise ist Ölsäure teuerer als Talgfettsäure, wobei die Ölsäure zur Herstellung der für die transluzenten oder klaren Weichmacherzusammensetzungen nötigen quartären Ammoniumverbindungen verwendet wird, während die Talgfettsäure bei der Herstellung der trüben Zusammensetzungen eingesetzt wird. Neben diesen beiden objektiven Vorteilen assoziiert der Verbraucher subjektiv zusätzliche Vorteile mit trüben Konditioniermitteln. Er erachtet ein trübes Konditioniermittel als ein besonders konzentriertes und damit wirkungsvolles, also werthaltiges Produkt, während ihm klare oder transluzente Mittel als wenig gehaltvoll erscheinen, da er die Trübheit des Mittels als Indikator für die Wirkstoffkonzentration heranzieht. Zudem sind ihm trübe Mittel seit langem als wirkungsvoll vertraut. Hinzu kommt noch das Faktum, das klare oder transluzente Mittel unter gegebenen Umständen eintrüben können oder sich in anderer Weise leicht optisch verändern können. Solche, wenn auch leichten, optischen Veränderungen führen schnell zu einer starken Verunsicherung des Verbrauchers, da dieser sich nicht im klaren ist, ob die Mittel noch sachgemäß eingesetzt werden können. Im Zweifelsfall verwendet der verunsicherte Verbraucher ein solches Mittel nach seiner optischen Veränderung nicht weiter. Bei trüben Mitteln sieht er sich mit diesen Problemen nicht konfrontiert, da hier leichte optische Veränderungen gar nicht wahrgenommen werden können.

Vor diesem Hintergrund ist es die Aufgabe der vorliegenden Erfindung, ein trübes frostresistentes Konditioniermittel bereitzustellen, daß auch bei Temperaturen unter 0°C nicht erstarrt, sondern einsatzfähig bleibt. Eine weitere Aufgabe der Erfindung ist es, daß ein solches trübes Konditioniermittel eine sehr gute Verteilbarkeit und hohe Auflösegeschwindigkeit in kaltem Wasser zeigt, auch nachdem es Temperaturen unter 0°C ausgesetzt worden ist. Eine zusätzliche Aufgabe der Erfindung ist es, daß ein solches trübes Konditioniermittel nicht nur frostresistent, sondern auch kältestabil ist. Dabei bedeutet der Begriff der Frostresistenz das Vermögen des Konditioniermittels bei Temperaturen unter 0°C nicht zu erstarren. Selbstverständlich ist die Frostresistenz nicht auf beliebig tiefe Temperaturen erstreckbar. Erstarrt das Mittel also bei Erreichen einer besonders tiefen Temperatur, beispielsweise bei -23°C, so bleibt es dennoch kältestabil, ist als nach dem Wiederauftauen verwendungsfähig und nicht infolge des Kälteeinflusses degeneriert, d. h. Produktfunktionalität und -konsistenz bleiben trotz Gefrier-/Auftau-Zyklen im wesentlichen erhalten.

Gegenstand der Erfindung ist daher in einer ersten Ausführungsform ein trübes frostresistentes, flüssiges, wässriges Konditioniermittel, enthaltend einen oder mehrere mehrwertige Alkohole und/oder Glycol ether als Cryoprotektoren, insbesondere in einer Menge größer 5 Gew.-%, bevorzugt größer 10 Gew.-%, weiter bevorzugt größer 15 Gew.-%, besonders bevorzugt größer 18 Gew.-%, besonders stark bevorzugt größer 20 Gew.-%, insbesondere größer 24 Gew.-% jeweils bezogen auf das gesamte Mittel, sowie wenigstens eine Weichmacherkomponente ausgewählt aus den nachfolgenden Formeln (a) : 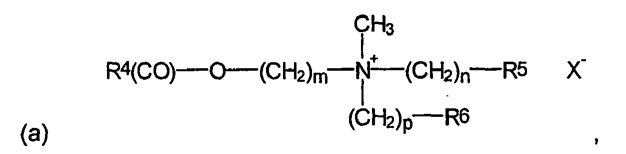 hierbei steht R⁴ für einen aliphatischen Alkylrest mit 12 bis 22 Kohlenstoffatomen mit 0, 1, 2 oder 3 Doppelbindungen; R⁵ steht für H, OH oder insbesondere O(CO)R⁷, R⁶ steht unabhängig von R⁵ für H, OH oder O(CO)R⁸, wobei R⁷ und R⁸ unabhängig voneinander jeweils für einen aliphatischen Alkylrest mit 12 bis 22 Kohlenstoffatomen mit 0, 1, 2 oder 3 Doppelbindungen steht, m, n und p können jeweils unabhängig voneinander den Wert 1, 2 oder 3 haben, X⁻ ist ein passendes Anion, vorzugsweise ein Halogenid-, Methosulfat-, Methophosphat- oder Phosphation sowie Mischungen aus diesen,
oder der Formel (b): 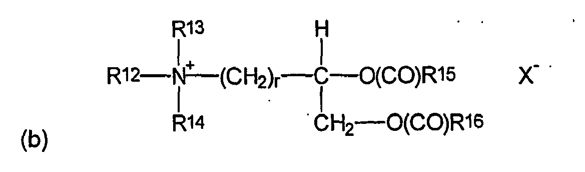 wobei R¹², R¹³ und R¹⁴ unabhängig voneinander für eine C₁₋₄-Alkyl-, Alkenyl-oder Hydroxyalkylgruppe steht, R¹⁵ und R¹⁶ jeweils unabhängig ausgewählt eine C₈₋₂₈-Alkylgruppe mit 0, 1, 2 oder 3 Doppelbindungen darstellt und r eine Zahl zwischen 0 und 5 ist, X⁻ ist ein passendes Anion, vorzugsweise ein Halogenid-, Methosulfat-, Methophosphat- oder Phosphation sowie Mischungen aus diesen.
sowie wenigstens ein Alkoholethoxylat mit wenigstens ZOEO Einheiten.

Bevorzugt sind Verbindungen, die für R⁴ und R⁷ Alkylreste mit 16 bis 18 Kohlenstoffatomen enthalten. Besonders bevorzugt sind Verbindungen, bei denen R⁶ zudem für OH steht. Beispiele für Verbindungen der Formel (a) sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Werden quarternierte Verbindungen der Formel (a) eingesetzt, die ungesättigte Alkylketten aufweisen, sind die Acylgruppen bevorzugt, deren korrespondierenden Fettsäuren eine Jodzahl zwischen 5 und 80, vorzugsweise zwischen 10 und 60 und insbesondere zwischen 30 und 50 aufweisen, sowie vorzugsweise ein cis/trans-Isomerenverhältnis (in Gew.-%) von größer als 30 : 70, vorzugsweise größer als 50 : 50 und insbesondere größer als 60 : 40 haben. Handelsübliche Beispiele sind die von Stepan unter dem Warenzeichen Stepantex^{®} vertriebenen Methylhydroxyalkyldialkoyloxyalkylammoniummethosulfate oder die unter Dehyquart^{®} bekannten Produkte von Cognis bzw. die unter Rewoquat^{®} bekannten Produkte von Degussa.

In einer bevorzugten Ausführungsform enthalten die erfindungsgemäßen Mittel quartäre Verbindungen gemäß den Formeln (a) oder (b), die ungesättigte Alkylketten aufweisen, wobei die korrespondierenden Fettsäuren eine Jodzahl zwischen 5 und 80, vorzugsweise zwischen 10 und 60 und insbesondere zwischen 30 und 50 aufweisen, sowie vorzugsweise ein cis/- trans-Isomerenverhältnis (in Gew.-%) von größer als 30:70, vorzugsweise größer als 50 : 50 und insbesondere größer als 60 : 40 haben. In einer weiteren bevorzugten Ausführungsform handelt es sich bei diesen korrespondierenden Fettsäuren um Talgfettsäure. Bis-(palmitoyl)-ethyl-hydroxyethyl-methyl-ammonium-methosulfat oder Methyl-N-(2-hydroxyethyl)-N,N-di(talgacyl-oxyethyl)ammonium-methosulfat sind besonders bevorzugte Weichmacherkomponenten.

Verbindungen der Formeln (a) und (b) sind sogenannte Esterquats. Esterquats zeichnen sich durch eine hervorragende biologische Abbaubarkeit aus, was ein wichtiger Vorteil im Sinne der Erfindung ist. Ein weiterer Vorteil der erfindungsgemäß enthaltenen Esterquats liegt in ihrer besonders vorteilhaften Verarbeitbarkeit sowie in ihrer besonders vorteilhaften Einarbeitbarkeit in die erfindungsgemäß herstellbaren Konditioniermittel, die an späterer Stelle näher beschrieben wird. Ein zusätzlicher Vorteil der erfindungsgemäß enthaltenen Esterquats liegt darin, daß sie in saurem Milieu besonders stabil sind. Hinzukommt als weiterer Vorteil der erfindungsgemäß enthaltenen Esterquats, daß sie stark zur Stabilität des gesamten Konditioniermittels beitragen. Vorteilhafterweise lassen sich daher sogar Konditioniermittel mit besonders hoher Konzentration an Weichmacherkomponente realisieren. Die vorgenannten Vorteile ergeben sich insbesondere im Vergleich zu herkömmlichen quartären Ammoniumverbindungen ohne die für Esterquats typischen Estergruppen oder im Vergleich zu quartären Ammoniumverbindungen, bei denen Alkylgruppen lediglich durch Amidogruppen unterbrochen sind. Bevorzugt im Sinne der Erfindung sind insbesondere Diesterquats, also solche quartären Ammoniumverbindungen, bei denen mindestens zwei Alkylgruppen durch jeweils eine Estergruppe unterbrochen ist.

Es wurde gefunden, daß, sich die erfindungsgemäßen Weichmacherkomponenten entsprechend den Formeln (a) und (b) auch in hohen Konzentrationen in das Konditioniermittel einarbeiten lassen, so daß also problemlos hochkonzentrierte, erfindungsgemäße Konditioniermittel darstellbar sind. Die erfindungsgemäßen hochkonzentrierten Konditioniermittel sind weiterhin frostresistent und kältestabil im Sinne der Erfindung. Hochkonzentrierte Konditioniermittel liegen dann vor, wenn der Gehalt an Weichmacherkomponente im Mittel bezogen auf das Mittel über 8 Gew.-%, bevorzugt über 10 Gew.-%, besonders bevorzugt über 12 Gew.-%, insbesondere über 15 Gew.-% liegt. Es ist ein sehr großer Vorteil, der erfindungsgemäßen Weichmacherkomponenten entsprechend den Formeln (a) und (b), daß diese sich in hohen Konzentrationen in das Konditioniermittel einarbeiten lassen, ohne daß es zu Problemen mit der Stabilität oder Frostresistenz des Mittels kommt.

In einer bevorzugten Ausführungsform enthalten die erfindungsgemäßen Mittel Weichmacherkomponenten in Mengen bis zu 25 Gew.-%, vorzugsweise von 0,1 bis 24 Gew.-%, besonders bevorzugt von 2 bis 22 Gew.-%, äußerst bevorzugt von 3 bis 20 Gew.-% und insbesondere von 5 bis 18 Gew.-%, jeweils bezogen auf das gesamte Mittel, wobei es sich bei der Weichmacherkomponente um eine Verbindung gemäß den Formeln a oder b handelt, insbesondere um einen Diesterquat gemäß einer dieser Formeln. Darüber hinaus kann das Mittel auch andere Weichmacherverbindungen enthalten.

Weitere bevorzugte Verbindungen sind die Diesterquats der Formel (I), die unter dem Namen Rewoquat® W 222 LM bzw. CR 3099 erhältlich sind und neben der Weichheit auch für Stabilität und Farbschutz sorgen.

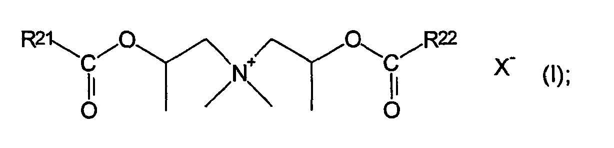

R²¹ und R²² stehen dabei unabhängig voneinander jeweils für einen aliphatischen Rest mit 12 bis 22 Kohlenstoffatomen mit 0, 1, 2 oder 3 Doppelbindungen.

In einer bevorzugten Ausführungsform zeichnet sich das Mittel dadurch aus, daß die Erstarrungstemperatur des Mittels nicht größer als -1°C, bevorzugt nicht größer als -5°C, besonders bevorzugt nicht größer als -10°C, insbesondere nicht größer als -15°ist.

Die die Weichmacherkomponente enthaltende wäßrige Zusammensetzung kann in prinzipieller Anlehnung an diverse, dem Fachmann wohlbekannte Weisen hergestellt werden. Solche wäßrige Zusammensetzungen werden beispielsweise durch Einrühren flüssiger Weichmacherkomponenten in Wasser und/oder einem organischen Lösungsmittel generiert. In Abhängigkeit von den Schmelzpunkten der Weichmacherkomponenten und der gegebenenfalls zugefügten weiteren Zusatzstoffe kann ein Erwärmen der wäßrigen Zusammensetzung angezeigt sein, beispielsweise auf Temperaturen zwischen 20 und 80°C, vorzugsweise auf über 40°C, insbesondere über 60°C.

Genauso gut können beispielsweise feste Weichmacherkomponenten und gegebenenfalls vorhandene Zusatzstoffe in Wasser und/oder organischem Lösungsmittel eingerührt werden. Vorzugsweise werden die festen Weichmacher geschmolzen - in der Regel auf Temperaturen zwischen 40 und 80°C- und anschließend unter intensivem Rühren in einem wäßrigen Medium dispergiert, welches zuvor gegebenenfalls temperiert worden ist, beispielsweise auf Temperaturen bis zu 80°C.

Die betreffenden wäßrigen Zusammensetzungen werden vorteilhafterweise intensiv gemischt, beispielsweise durch Rührung. Die Einarbeitung von Cryoprotektoren in die entsprechenden wäßrigen Zubereitungen erfolgt erfindungsgemäß vorteilhafterweise erst dann, wenn die weichmachende Komponente bereits in der wäßrigen Zubereitung enthalten ist.

Unter dem Begriff des Cryoprotektors versteht man im Sinne der Erfindung im weitesten Sinne alle jene Substanzen oder Substanzgemische gemäß Anspruch 1 die, wenn sie in erfindungsgemäße Konditioniermittel eingearbeitet werden, dazu führen, daß die Erstarrungstemperatur des betreffenden Konditioniermittels auf eine Temperatur von mindestens -1 °C, bevorzugt von mindestens -5°C, besonders bevorzugt von mindestens -10°, ganz besonders bevorzugt von mindestens -15°C abgesenkt wird.

Vorteilhafterweise können erfindungsgemäße Konditioniermittel als Folge dieser deutlichen Absenkung der Erstarrungstemperatur nun bei viel tieferen Temperaturen gelagert werden, ohne daß sie erstarren und infolgedessen gegebenenfalls unbrauchbar werden. Auf diese Weise werden unerwünschte Veränderungen des Konditioniermittels als Resultat seiner Erstarrung infolge Aufbewahrung bei tiefen Temperaturen verhindert.

Hinzu kommt, daß erfindungsgemäße frostresistente Konditioniermittel selbst dann, wenn sie doch unter ihren Erstarrungstemperatur abgekühlt werden sollten, auch nach dem Wiederauftauen noch verwendbar und stabil sind. Das bedeutet, daß die Mittel sowohl frostresistent als auch kältestabil sind. Mehrfaches Einfrieren und Auftauen führt hier also nicht zu einer Degeneration des Konditioniermittels im Sinne eines Verlustes der Funktionalität des Mittels infolge möglicher Ausfällungen, Phasenseparationen oder Vergelungen etc. Auch die Konsistenz des Mittels bleibt im wesentlichen unverändert.

Das erfindungsgemäße Mittel zeichnet sich dadurch aus, daß es sich bei den Cryoprotektoren um Substanzen ausgewählt aus der Gruppe der mehrwertigen Alkohole und/oder Glycolether und/oder deren Gemische, insbesondere aber um Dipropylenglykol handelt.

Zu anderen typischen als Cryoprotektor verwendbaren mehrwertigen Alkoholen bzw. deren Derivaten zählen beispielsweise Propylenglykol, Ethylenglykol oder Diethylenglykol und so weiter, sowie Mischungen solcher mehrwertigen Alkohole bzw. Glykolether. Eine überaus vorteilhafte Substanz im Sinne der Erfindung ist aber Dipropylenglykol. Wie die Anmelderin überraschend finden konnte, zeichnet sich das Dipropylenglykol in den erfindungsgemäßen Mitteln zusätzlich dadurch aus, daß es im Stande ist, hydrophobe Parfums, die gegebenenfalls in den Konditioniermitteln enthalten sein können, zu stabilisieren. Das Dipropylenglykol erfüllt also eine Doppelfunktion.

Eine zusätzliche Aufgabe dieser Erfindung ist es, trübe frostresistente Konditioniermittel bereitzustellen, die eine Parfumstabilisierung ermöglichen, so daß also parfumhaltige, frostresistente Mittel im Sinne der Erfindung bereitstellbar sind. Dabei bezieht sich der Terminus der Parfumstabilisierung auf hydrophobe Parfumöle, also solche Parfumöle, die im wesentlichen nicht wasserlöslich sind, wie beispielsweise bestimmte Aldehyde oder Ester. Bei Anwesenheit von hydrophoben Parfumölen darf es nicht zu einer Bildung von Öltröpfchen oder Phasenseparationen kommt. Die Parfumstabilisierung muß bei Raumtemperatur, bei tiefen Temperaturen im Sinne dieser Erfindung sowie nach dem Durchlaufen von Gefrier-/Auftau-Zyklen gegeben sein, so daß in allen drei Fällen beispielsweise keine Phasenseparationen auftreten.

In einer bevorzugten Ausführungsform enthält das erfindungsgemäße Mittel ein oder mehrere Parfums, insbesondere hydrophobe Parfums, vorzugsweise in einer Menge von bis zu 3 Gew.-%, bevorzugt in Mengen von 0,01 bis 1,4 Gew.-%, weiter bevorzugt 0,05 bis 1,0 Gew.-%, jedoch besonders bevorzugt 0,1 bis 0,8 Gew.-% und äußerst bevorzugt 0,2 bis 0,7 Gew.-%.

Als Parfumöle bzw. Duftstoffe können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfumöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Überraschenderweise konnte die Anmelderin finden, daß die erfindungsgemäßen Weichmacherverbindungen, d. h. die Esterquats der Formeln (a) und (b) im Hinblick auf die zu leistende Parfumstabilisierung hydrophober Parfums als vorteilhaft einzustufen sind, besonders wenn man sie mit herkömmlichen quartären Ammoniumverbindungen ohne die für Esterquats typischen Estergruppen oder mit quartären Ammoniumverbindungen, bei denen Alkylgruppen lediglich durch Amidogruppen unterbrochen sind, vergleicht. Es sei hier ausdrücklich angemerkt, daß die erfindungsgemäßen Mittel selbstverständlich auch hydrophile, also vorwiegend wasserlösliche Parfums beinhalten können. Hydrophile Parfums sind jedoch im Hinblick auf die Stabilität des resultierenden Mittels weniger problematisch.

Um die erfindungsgemäße Parfumstabilisierung hydrophober Parfums aber letztlich zu gewährleisten werden bestimmte Alkoholethoxylate eingesetzt.

Daher enthält das Mittel in wenigstens ein Alkoholethoxylat mit wenigstens 20 vorzugsweise wenigstens 30 EO-Einheiten. Zu den Alkoholethoxylaten zählen ethoxylierte insbesondere primäre Alkohole mit vorzugsweise 8 bis 18 C-Atomen und durchschnittlich mehr als 20 insbesondere mehr als 30 Mol Ethylenoxid (EO) pro Mol Alkohol, eingesetzt. Besonders bevorzugt sind C₈-C₁₆-Alkoholethoxylate, vorteilhafterweise C₁₀-C₁₅-Alkoholethoxylate, insbesondere C₁₂-C₁₄-Alkoholethoxylate, mit einem Ethoxylierungsgrad über 20 insbesondere über 30, vorzugsweise über 35. Der Alkoholrest kann vorzugsweise linear oder besonders bevorzugt in 2-Stellung methylverzweigt sein bzw. lineare und methylverzweigte Reste im Gemisch enthalten, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 40 EO pro Mol Alkohol bevorzugt. Zu den bevorzugten ethoxylierten Alkoholen gehören beispielsweise C₁₂₋₁₄-Alkohole mit ca. 40 EO, C₉₋₁₁-Alkohol mit ca. 40 EO, C₁₃₋₁₅-Alkohole mit ca. 40 EO, C₁₂₋₁₈-Alkohole mit ca. 40 EO und Mischungen aus diesen. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates NRE)auf.

Es können auch Fettalkohole mit deutlich mehr als 20 oder 30 oder 40 EO eingesetzt werden. Beispiele hierfür sind Talgfettalkohol mit 100 EO oder 120 EO. Der Vorteil der hochethoxylierter Fettalkohole, d. h. solcher mit EO > 30, liegt darin, daß sie sehr gute Parfumemulgiermittel sind, so daß es bei Anwesenheit von hydrophoben Parfumölen nicht zu einer Bildung von Öltröpfchen oder Phasenseparationen kommt.

Besonders bevorzugt sind ethoxylierte Alkohole mit einem Ethoxylierungsgrad von ca. 40 EO oder größer, insbesondere Rizinusöle mit einem Ethoxylierungsgrad von ca. 40 EO oder größer. Rizinusöl besteht zu 80-85% aus dem Glycerid der Ricinolsäure, sowie aus Glyceriden der Ölsäure (7%), Linolsäure (3%), Palmitinsäure (2%) u. Stearinsäure (1%).

In einer bevorzugten Ausführungsform handelt es sich bei dem Alkoholethoxylat um ein hochethoxyliertes Rizinusöl mit wenigstens 20 insbesondere wenigstens 30 EO-Einheiten, insbesondere um ein Rizinusöl mit einem Ethoxylierungsgrad von ca. 40 EO oder größer.

In einer bevorzugten Ausführungsform weist das herzustellende Mittel einen Gesamtgehalt an Alkoholethoxylat, jeweils bezogen auf das Mittel, von unter 10 Gew.-%, vorzugsweise unter 5 Gew.-%, bevorzugt unter 1 Gew.-%, insbesondere aber zwischen 0,1 bis 0,6 Gew.-% , weiter bevorzugt zwischen 0,15 bis 0,5 Gew.-%, jedoch besonders bevorzugt zwischen 0,2 bis 0,4 Gew.-% auf.

Die Anmelderin konnte bezüglich der erfindungsgemäßen Alkoholethxylate finden, daß durch diese eine Parfumstabilisierung bei Raumtemperatur, bei tiefen Temperaturen im Sinne dieser Erfindung sowie nach dem Durchlaufen von Gefrier-/Auftau-Zyklen gegeben ist, so daß in allen drei Fällen beispielsweise keine Phasenseparationen auftreten. Der Terminus der Parfumstabilisierung bezieht sich dabei auf hydrophobe Parfums im vorgenannten Sinne. Die Anmelderin geht davon aus, ohne sich an eine bestimmte Theorie zu binden, daß die erfindungsgemäßen Weichmacherverbindungen, d. h. die Esterquats der Formeln (a) und (b) die Funktion der erfindungsgemäßen Alkoholethxylate hinsichtlich deren Parfumstabilisierung positiv beeinflussen, da beispielsweise bei ausschließlicher Verwendung von herkömmlichen quartären Ammoniumverbindungen ohne die für Esterquats typischen Estergruppen oder von quartären Ammoniumverbindungen, bei denen Alkylgruppen lediglich durch Amidogruppen unterbrochen sind, schlechtere Ergebnisse von der Anmelderin beobachtet werden konnten, was die Stabilität der Konditioniermittel, insbesondere die Parfumstabilisierung betrifft.

Die Anmelderin hat überraschenderweise gefunden, daß eine Vielzahl von Verbindungen, die in anderen Bereichen zur Senkung des Gefrierpunktes flüssiger Mittel erfolgreich eingesetzt werden können, im Sinne der Erfindung nicht als cryoprotektive Substanzen eingesetzt werden können, um frostresistente, ggf. parfumhaltige Konditioniermittel zu erhalten. Beispielsweise führt der Einsatz von Isopropanol oder Ethanol an Stelle der verwendeten erfindungsgemäß einsetzbaren Cryoprotektoren zu einer puddingartigen Konsistenz der resultierenden, parfumhaltigen Konditioniermittel. Ebenso führt die Verwendung von Salzen organischer Säuren wie beispielsweise von Natriumcitrat, Kaliumformiat oder Kaliumacetet ebenfalls zu unerwünschten Konsistenzveränderungen. Auch Stärkederivate sowie Aminoessigsäure zeigten nicht den erfindungsgemäßen Erfolg.

Als zusätzliche Weichmacherkomponenten neben den erfindungsmäßig enthaltenen Esterquats können die erfindungsgemäßen Konditioniermittel auch weitere weichmachende Komponenten, wie beispielsweise konventionelle quartäre Ammoniumverbindungen, kationische Polymere und Emulgatoren, wie sie in Haarpflegemitteln und auch in Mitteln zur Textilavivage eingesetzt werden, enthalten.

Geeignete Beispiele sind quartäre Ammoniumverbindungen der Formel (II), 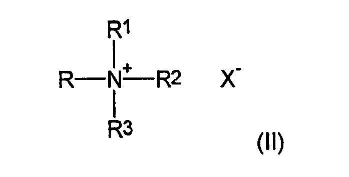 wobei in (II) R und R¹ für einen acyclischen Alkylrest mit 12 bis 24 Kohlenstoffatomen, R² für einen gesättigten C₁-C₄ Alkyl- oder Hydroxyalkylrest steht, R³ entweder gleich R, R¹ oder R² ist oder für einen aromatischen Rest steht. X⁻ steht entweder für ein Halogenid-, Methosulfat-, Methophosphat- oder Phosphation sowie Mischungen aus diesen. Beispiele für kationische Verbindungen der Formel (II) sind Didecyldimethylammoniumchlorid, Ditalgdimethylammoniumchlorid oder Dihexadecylammoniumchlorid.

Neben den erfindungsgemäßen quartären Verbindungen können beispielsweise auch quartäre Imidazoliniumverbindungen der Formel (III) eingesetzt werden, 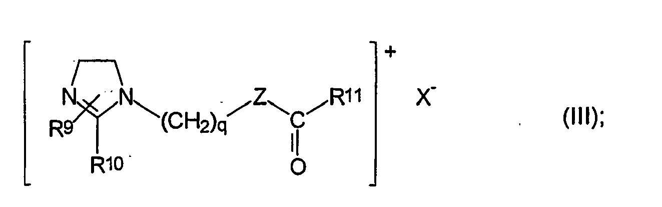 wobei R⁹ für H oder einen gesättigten Alkylrest mit 1 bis 4 Kohlenstoffatomen, R¹⁰ und R¹¹ unabhängig voneinander jeweils für einen aliphatischen, gesättigten oder ungesättigten Alkylrest mit 12 bis 18 Kohlenstoffatomen, R¹⁰ alternativ auch für O(CO)R²⁰ stehen kann, wobei R²⁰ einen aliphatischen, gesättigten oder ungesättigten Alkylrest mit 12 bis 18 Kohlenstoffatomen bedeutet, und Z eine NH-Gruppe oder Sauerstoff bedeutet und X⁻ ein Anion ist. q kann ganzzahlige Werte zwischen 1 und 4 annehmen.

Neben den erfindungsgemäßen Verbindungen der Formeln (a) und (b) können auch kurzkettige, wasserlösliche, quartäre Ammoniumverbindungen eingesetzt werden, wie Trihydroxyethylmethylammonium-methosulfat oder die Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid.

Auch protonierte Alkylaminverbindungen, die weichmachende Wirkung aufweisen, sowie die nicht quaternierten, protonierten Vorstufen der kationischen Emulgatoren sind geeignet, um neben den erfindungsgemäßen Verbindungen der Formeln (a) und (b) eingesetzt zu werden.

Weitere neben den erfindungsgemäßen Verbindungen der Formeln (a) und (b) einsetzbaren Verbindungen weichmachender Wirkung stellen die quaternisierten Proteinhydrolysate dar.

Zu den geeigneten kationischen Polymeren, die neben den erfindungsgemäßen Verbindungen der Formeln (a) und (b) eingesetzt werden können, zählen die Polyquaternium-Polymere, wie sie im CTFA Cosmetic Ingredient Dictionary (The Cosmetic, Toiletry und Fragrance, Inc., 1997), insbesondere die auch als Merquats bezeichneten Polyquaternium-6-, Polyquatemium-7-, Polyquaternium-10-Polymere (Ucare Polymer IR 400; Amerchol), Polyquaternium-4-Copolymere, wie Pfropfcopolymere mit einem Cellulosegerüst und quartären Ammoniumgruppen, die über Allyldimethylammoniumchlorid gebunden sind, kationische Cellulosederivate, wie kationisches Guar, wie Guar-hydroxypropyltriammoniumchlorid, und ähnliche quaternierte Guar-Derivate (z. B. Cosmedia Guar, Hersteller: Cognis GmbH), kationische quartäre Zuckerderivate (kationische Alkylpolyglucoside), z. B. das Handelsprodukt Glucquat^{®}100, gemäß CTFA-Nomenklatur ein "Lauryl Methyl Gluceth-10 Hydroxypropyl Dimonium Chloride", Copolymere von PVP und Dimethylaminomethacrylat, Copolymere von Vinylimidazol und Vinylpyrrolidon, Aminosilicon-polymere und Copolymere.

Ebenfalls neben den erfindungsgemäßen Verbindungen der Formeln (a) und (b) sind polyquaternierte Polymere (z. B. Luviquat Care von BASF) und auch kationische Biopolymere auf Chitinbasis und deren Derivate, beispielsweise das unter der Handelsbezeichnung Chitosan^{®} (Hersteller: Cognis) erhältliche Polymer einsetzbar.

Erfindungsgemäß ebenfalls geeignet, um neben den erfindungsgemäßen Verbindungen der Formeln (a) und (b) eingesetzt zu werden, sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) Abil^{®}-Quat 3270 und 3272 (Hersteller: Goldschmidt-Rewo; diquartäre Polydimethylsiloxane, Quaternium-80), sowie Siliconquat Rewoquat^{®} SQ 1 (Tegopren^{®} 6922, Hersteller: Degussa).

Ebenfalls einsetzbar neben den erfindungsgemäßen Verbindungen der Formeln (a) und (b) sind Verbindungen der Formel (IV), 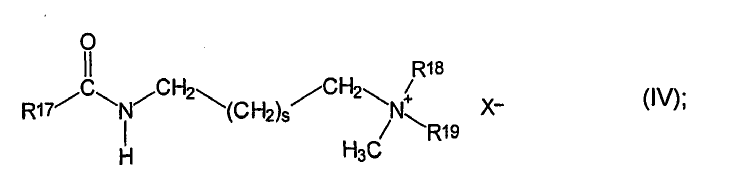 die Alkylamidoamine in ihrer nicht quaternierten oder, wie dargestellt, ihrer quaternierten Form, sein können. R¹⁷ kann ein aliphatischer Alkylrest mit 12 bis 22 Kohlenstoffatomen mit 0, 1, 2 oder 3 Doppelbindungen sein. s kann Werte zwischen 0 und 5 annehmen. R¹⁸ und R¹⁹ stehen unabhängig voneinander jeweils für H, C₁₋₄-Alkyl oder Hydroxyalkyl. Bevorzugte Verbindungen sind Fettsäureamidoamine wie das unter der Bezeichnung Tego Amid^{®}S 18 erhältliche Stearylamidopropyldimethylamin oder das unter der Bezeichnung Stepantex^{®} X 9124 erhältliche 3-Talgamidopropyl-trimethylammonium-methosulfat, die sich neben einer guten konditionierenden Wirkung auch durch farbübertragungsinhibierende Wirkung sowie speziell durch ihre gute biologische Abbaubarkeit auszeichnen. Besonders bevorzugt sind alkylierte quaternäre Ammoniumverbindungen, von denen mindestens eine Alkylkette durch eine Estergruppe und/oder Amidogruppe unterbrochen ist, insbesondere N-Methyl-N(2-hydroxyethyl)-N,N-(ditalgacyloxyethyl)ammonium-methosulfat und/oder N-Methyl-N(2-hydroxyethyl)-N,N-(palmitoyloxyethyl)ammonium-methosulfat.

In einer weiteren bevorzugten Ausführungsform enthalten die erfindungsgemäß herzustellenden Mittel zusätzlich gegebenenfalls Elektrolyte. Elektrolyte können beispielsweise der Viskositätsregulation (Viskositätsregulator) dienen und können üblicherweise in Mengen bis zu 15 Gew.-%, vorzugsweise bis zu 10 Gew.-%, besonders bevorzugt von 0,5 bis 8 Gew.-% und insbesondere von 1 bis 6 Gew.-%, jeweils bezogen auf das gesamte Mittel, eingesetzt werden.

Als Elektrolyte können anorganische Salze eingesetzt werden. Bevorzugte Kationen sind dabei die Alkali- und Erdalkalimetalle, bevorzugte Anionen sind die Halogenide und Sulfate. Von besonderer Vorteilhaftigkeit ist dabei der Einsatz von MgCl₂, vorzugsweise Magnesiumchloridhexahydrat in den erfindungsgemäßen Mitteln.

In einer bevorzugten Ausführungsform enthält das erfindungsgemäße Mittel anorganische Salze, insbesondere Magnesiumchlorid, vorzugsweise Magnesiumchloridhexahydrat. Sollen bestimmte Mindestmengen an Cryprotektor und/oder Weichmacherverbindung in das erfindungsgemäß herzustellende Konditioniermittel eingearbeitet werden, so stellt das Hinfügen von MgCl₂, insbesondere von MgCl₂x6H₂O oder einer vergleichbaren Substanz in den Reaktionsansatz eine besonders bevorzugte Ausführungsform dar. Soll die Endzusammensetzung des herzustellenden Konditioniermittels mehr als 15 Gew.-% an Cryoprotektor, beispielsweise Dipropylenglykol, und/oder mehr als 7 Gew.-% einer Weichmacherverbindung enthalten, so stellt die Zugabe von MgCl₂, insbesondere von MgCl₂x6H₂O bei der Herstellung des Konditioniermittels eine besonders bevorzugte Ausführungsform dar.

Vorteilhafterweise enthält ein solches Mittel mindestens 0,005 Gew.-%, bevorzugt mindestens 0,01 Gew.-%, insbesondere 0,02 Gew.-% MgCl₂x6H₂O, bezogen auf das gesamte Mittel . Die zuzugebende Menge an MgCl₂x6H₂O soll 8 Gew.-%, bezogen auf das Mittel nicht überschreiten, bevorzugt soll die Menge nicht größer als 6 Gew.-% sein, insbesondere nicht größer als 4 Gew.-% sein.

Als besonders vorteilhaft für die Stabilität, Kältestabilität und Frostresistenz des erfindungsgemäß herstellbaren Konditioniermittels hat es sich zudem erwiesen, zusätzlich eine zwitterionische Verbindung nachstehender Formel V in Mengen vorzugsweise bis zu 10 Gew.-% in das herzustellende Mittel einzubringen:

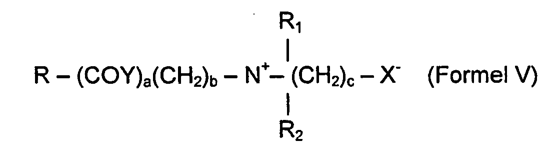

Hierbei steht R für eine C₆₋₂₈-Alkyl- oder Alkenylgruppe; R₁ und R₂ sind jeweils, unabhängig voneinander, C₁₋₄-Alkyl-Gruppen; a steht für die Zahl 0 oder 1, b und c sind jeweils, unabhängig voneinander, aus ganzen Zahlen von 1 bis 4 ausgewählt; Y ist Sauerstoff; X ist ein kompatibles Anion. Bei entsprechendem pH-Wert kann das Anion protoniert sein. In einer bevorzugten Ausführungsform zeichnet sich daher das erfindungsgemäße Mittel dadurch aus, daß es eine zwitterionische Verbindung der Formel V in Mengen bis zu 10 Gew.-% zusätzlich enthält.

Das zwitterionische Material kann auch ein Alkylamidoalkylendimethylaminocarbonsäure-Betain sein, mit der Formel VI: 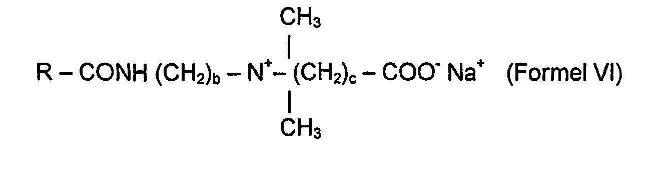 wobei b und c, unabhängig voneinander, ganze Zahlen von 1-4 sind, vorzugsweise b=2 oder 3 und c=2 oder 3 und wobei R für eine C₁₀₋₁₈-Alkylkette oder Mischungen davon steht.

In einer bevorzugten Ausführungsform zeichnet sich daher das erfindungsgemäße Mittel dadurch aus, daß es sich bei der zwitterionischen Verbindung um ein Alkylamidoalkylendimethylaminocarbonsäure-Betain der Formel **VI** handelt.

Das erfindungsgemäße Mittel liegt in einer bevorzugten Ausführungsform als Dispersion vor und weist eine mittlere Partikelgröße von kleiner 500 µm, besonders bevorzugt von kleiner 300 µm, äußerst bevorzugt von kleiner 200 µm auf, insbesondere liegt die mittlere Partikelgröße zwischen 0,005 µm und 180 µm.

Das erfindungsgemäße Mittel kann weitere Zusatz- und Hilfsstoffe enthalten, die im Verlauf des Herstellungsverfahrens zugesetzt werden können. Die Auswahl der Hilfs- und Zusatzstoffe bereitet dem Fachmann keinerlei Schwierigkeiten und wird so gewählt, daß die frostresistenten Eigenschaften der Mittel gewahrt bleiben.

In einer bevorzugten Ausführungsform kann das erfindungsgemäße Konditioniermittel Fettsäuren enthalten, insbesondere solche mit einer Iodzahl von 0 bis 25, aber auch solche mit höheren Iodzahlen. Beispielsweise handelt es sich dabei um Fettsäuren ausgewählt aus der folgender Gruppe der Laurin-, Tridecan-, Myristin-, Pentadecan-, Palmitin-, Margarin-, Stearin-, Nonadecan-, Arachin-, Behen-, Lignocerin-, Cerotin-, Melissinsäure, Palmitolein-, Öl-, Erucasäure, Linol-, Linolen-, Elaeostearin-, Arachidonsäure.

In einer bevorzugten Ausführungsform beträgt das Gewichtsverhältnis von Weichmacherkomponente zu Fettsäure 25 : 1 bis 5 : 1.

Als weitere Tenside, die zusätzlich in den erfindungsgemäßen Mitteln enthalten sein können, kommen sogenannte Gemini-Tenside in Betracht. Hierunter werden im allgemeinen solche Verbindungen verstanden, die zwei hydrophile Gruppen und zwei hydrophobe Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, daß die hydrophilen Gruppen einen ausreichenden Abstand haben, damit sie unabhängig voneinander agieren können. Derartige Tenside zeichnen sich im allgemeinen durch eine ungewöhnlich geringe kritische Micellkonzentration und die Fähigkeit, die Oberflächenspannung des Wassers stark zu reduzieren, aus. In Ausnahmefällen werden jedoch unter dem Ausdruck Gemini-Tenside nicht nur dimere, sondern auch trimere Tenside verstanden.

Geeignete Gemini-Tenside sind beispielsweise sulfatierte Hydroxymischether gemäß der deutschen Patentanmeldung DE-A-43 21 022 oder Dimeralkohol-bis- und Trimeralkohol-trissulfate und -ethersulfate gemäß der internationalen Patentanmeldung WO-A-96/23768**.** Endgruppenverschlossene dimere und trimere Mischether gemäß der deutschen Patentanmeldung DE-A-195 13 391 zeichnen sich insbesondere durch ihre Bi- und Multifunktionalität aus. So besitzen die genannten endgruppenverschlossenen Tenside gute Netzeigenschaften und sind dabei schaumarm, so daß sie sich insbesondere für den Einsatz in maschinellen Wasch- oder Reinigungsverfahren eignen.

Eingesetzt werden können aber auch Gemini-Polyhydroxyfettsäureamide oder Poly-Polyhydroxyfettsäureamide, wie sie in den internationalen Patentanmeldungen WO-A-95/19953**,** WO-A-95/19954 und WO-A-95/19955 beschrieben werden.

Weitere geeignete Tenside sind Polyhydroxyfettsäureamide der folgenden Formel, 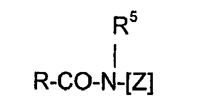 in der RCO für einen aliphatischen Acylrest mit 6 bis 22 Kohlenstoffatomen, R⁵ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der folgenden Formel, 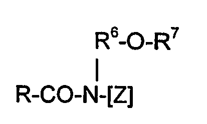 in der R für einen linearen oder verzweigten Alkyl- oder Alkenylrest mit 7 bis 12 Kohlenstoffatomen, R⁶ für einen linearen, verzweigten oder cyclischen Alkylrest oder einen Arylrest mit 2 bis 8 Kohlenstoffatomen und R⁷ für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können dann beispielsweise nach der Lehre der internationalen Anmeldung WO-A-95/07331 durch Umsetzung mit Fettsäuremethylestern in Gegenwart eines Alkoxids als Katalysator in die gewünschten Polyhydroxyfettsäureamide überführt werden.

Um den pH-Wert der erfindungsgemäßen Mittel in den gewünschten Bereich zu bringen, kann der Einsatz von pH-Stellmitteln angezeigt sein. Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet. Üblicherweise überschreitet die Menge dieser Stellmittel 2 Gew.-% der Gesamtformulierung nicht.

Das erfindungsgemäße Mittel weist bei einer Temperatur von 20°C einen pH-Wert von 1 bis 7, vorzugsweise von 1,5 bis 5 und insbesondere von 2,0 bis 4,0 auf.

In einer bevorzugten Ausführungsform liegt der pH-Wert des erfindungsgemäßen Mittels bei einer Temperatur von 20°C zwischen 2, 0 und 4,0.

Neben geringeren Mengen von anionischen Tensiden können die erfindungsgemäßen Mittel gegebenenfalls weitere übliche Hilfs- und Zusatzstoffe, insbesondere aus der Gruppe der Gerüststoffe, Enzyme, Bleichmittel, Bleichaktivatoren, Komplexbildner, Duftstoffe, Parfumträger, Fluoreszenzmittel, Farbstoffe, Verdickungsmittel, Schauminhibitoren, Vergrauungsinhibitoren, Knitterschutzmittel, antimikrobielle Wirkstoffe, Germizide, Fungizide, Antioxidantien, Antistatika, UV-Absorber, optischen Aufheller, Antiredepositionsmittel, Perlglanzgeber, Farbübertragungsinhibitoren, Einlaufverhinderer, Korrosionsinhibitoren, Konservierungsmittel, Phobier- und Imprägniermittel, Hydrotrope sowie Quell- und Schiebefestmittel enthalten.

In einer bevorzugten Ausführungsform kann das erfindungsgemäße Mittel gegebenenfalls zusätzlich ein oder mehrere Komplexbildner enthalten.

Komplexbildner (INCI Chelating Agents), auch Sequestriermittel genannt, sind Inhaltsstoffe, die Metallionen zu komplexieren und inaktivieren vermögen, um ihre nachteiligen Wirkungen auf die Stabilität oder das Aussehen der Mittel, beispielsweise Trübungen, zu verhindern. Einerseits ist es dabei wichtig, die mit zahlreichen Inhaltsstoffen inkompatiblen Calcium- und Magnesiumionen der Wasserhärte zu komplexieren. Die Komplexierung der Ionen von Schwermetallen wie Eisen oder Kupfer verzögert die oxidative Zersetzung der fertigen Mittel.

Geeignet sind beispielsweise die folgenden gemäß INCI bezeichneten Komplexbildner, die im *International Cosmetic Ingredient Dictionary and Handbook* näher beschrieben sind: Aminotrimethylene Phosphonic Acid, Beta-Alanine Diacetic Acid, Calcium Disodium EDTA, Citric Acid, Cyclodextrin, Cyclohexanediamine Tetraacetic Acid, Diammonium Citrate, Diammonium EDTA, Diethylenetriamine Pentamethylene Phosphonic Acid, Dipotassium EDTA, Disodium Azacycloheptane Diphosphonate, Disodium EDTA, Disodium Pyrophosphate, EDTA, Etidronic Acid, Galactaric Acid, Gluconic Acid, Glucuronic Acid, HEDTA, Hydroxypropyl Cyclodextrin, Methyl Cyclodextrin, Pentapotassium Triphosphate, Pentasodium Aminotrimethylene Phosphonate, Pentasodium Ethylenediamine Tetramethylene Phosphonate, Pentasodium Pentetate, Pentasodium Triphosphate, Pentetic Acid, Phytic Acid, Potassium Citrate, Potassium EDTMP, Potassium Gluconate, Potassium Polyphosphate, Potassium Trisphosphonomethylamine Oxide, Ribonic Acid, Sodium Chitosan Methylene Phosphonate, Sodium Citrate, Sodium Diethylenetriamine Pentamethylene Phosphonate, Sodium Dihydroxyethylglycinate, Sodium EDTMP, Sodium Gluceptate, Sodium Gluconate, Sodium Glycereth-1 Polyphosphate, Sodium Hexametaphosphate, Sodium Metaphosphate, Sodium Metasilicate, Sodium Phytate, Sodium Polydimethylglycinophenolsulfonate, Sodium Trimetaphosphate, TEA-EDTA, TEA-Polyphosphate, Tetrahydroxyethyl Ethylenediamine, Tetrahydroxypropyl Ethylenediamine, Tetrapotassium Etidronate, Tetrapotassium Pyrophosphate, Tetrasodium EDTA, Tetrasodium Etidronate, Tetrasodium Pyrophosphate, Tripotassium EDTA, Trisodium Dicarboxymethyl Alaninate, Trisodium EDTA, Trisodium HEDTA, Trisodium NTA und Trisodium Phosphate.

Bevorzugte Komplexbildner sind tertiäre Amine, insbesondere tertiäre Alkanolamine (Aminoalkohole). Die Alkanolamine besitzen sowohl Amino- als auch Hydroxy- und/oder Ethergruppen als funktionelle Gruppen. Besonders bevorzugte tertiäre Alkanolamine sind Triethanolamin und Tetra-2-hydroxypropylethylendiamin (N,N,N',N'-Tetrakis-(2-hydroxy-propyl)ethylendiamin).

Ein besonders bevorzugter Komplexbildner ist die Etidronsäure (1-Hydroxyethyliden-1,1-diphosphonsäure, 1-Hydroxyethyan-1,1-diphosphonsäure, HEDP, Acetophosphonsäure, INCI Etidronic Acid) einschließlich ihrer Salze. In einer bevorzugten Ausführungsform enthält das erfindungsgemäße Mittel demgemäß als Komplexbildner Etidronsäure und/oder eines oder mehrere ihrer Salze.

In einer besonderen Ausführungsform kann das erfindungsgemäße Mittel eine Komplexbildnerkombination aus einem oder mehreren tertiären Aminen und einer oder mehreren weiteren Komblexbildnern, vorzugsweise einer oder mehreren Komplexbildnersäuren oder deren Salzen, insbesondere aus Triethanolamin und/oder Tetra-2-hydroxypropylethylendiamin und Etidronsäure und/oder einem oder mehrerer ihrer Salze enthalten.

Das Mittel enthält gegebenenfalls Komplexbildner in einer Menge von üblicherweise 0 bis 20 Gew.-%, vorzugsweise 0,1 bis 15 Gew.-%, insbesondere 0,5 bis 10 Gew.-%, besonders bevorzugt 1 bis 8 Gew.-%, äußerst bevorzugt 1,5 bis 6 Gew.-%, beispielsweise 1,5, 2,1, 3 oder 4,2 Gew.-% enthalten.

In einer weiteren Ausführungsform enthält das Mittel gegebenenfalls ein oder mehrere Verdickungsmittel.

Die Viskosität der gegebenenfalls flüssigen Mittel kann mit üblichen Standardmethoden (beispielsweise Brookfield-Viskosimeter Typ RV bei 20 U/min und 20°C, Spindel 1) gemessen werden und liegt vorzugsweise im Bereich von 3 bis 5000 mPas. Bevorzugte flüssige bis gelförmige Mittel haben Viskositäten von 10 bis 4000 mPas, wobei Werte zwischen 20 und 2000 mPas besonders bevorzugt sind. Idealerweise liegt die Viskosität eines erfindungsgemäßen Konditioniermittels unmittelbar nach der Herstellung bei ca. 25 mPas.

Geeignete Verdicker sind anorganische oder polymere organische Verbindungen. Es können auch Gemische aus mehreren Verdickern eingesetzt werden. Als Verdicker können dem Fachmann geläufige Mittel eingesetzt werden. Der Fachmann wählt dazu insbesondere solche Mittel aus, die unter den bestimmungsgemäßen Bedingungen eine ausreichende Stabilität, insbesondere gute Stabilität in einem saurem pH-Wertbereich aufweisen. Beispiele für Verdicker, die in flüssigen Formulierungen Anwendung finden, sind:

Zu den anorganischen Verdickern zählen beispielsweise Polykieselsäuren, Tonmineralien wie Montmorillonite, Zeolithe, Kieselsäuren, Aluminiumsilikate, Schichtsilikate und Bentonite. Die organischen Verdicker, die auch gleichzeitig als Stabilisator wirken können, stammen aus den Gruppen der natürlichen Polymere, der abgewandelten natürlichen Polymere und der vollsynthetischen Polymere.

Aus der Natur stammende Polymere, die als Verdicker Verwendung finden, sind beispielsweise Xanthan, Agar-Agar, Carrageen, Tragant, Gummi arabicum, Alginate, Pektine, Polyosen, Guar-Mehl, Gellan-Gum, Johannisbrotbaumkernmehl, Stärke, Dextrine, Gelatine und Casein.

Abgewandelte Naturstoffe stammen vor allem aus der Gruppe der modifizierten Stärken und Cellulosen, beispielhaft seien hier Carboxymethylcellulose und andere Celluloseether, Hydroxyethyl- und -propylcellulose, hochveretherte Methylhydroxyethylcellulose sowie Kernmehlether genannt.

Eine große Gruppe von Verdickern, die breite Verwendung in den unterschiedlichsten Anwendungsgebieten finden, sind die vollsynthetischen Polymere wie Polyacryl- und Polymethacryl-Verbindungen, die vernetzt oder unvernetzt und ggf. kationisch modifiziert sein können, Vinylpolymere, Polycarbonsäuren, Polyether, aktivierte Polyamidderivate, Rizinusölderivate, Polyimine, Polyamide und Polyurethane. Beispiele für derartige Polymer sind Acrylharze, Ethylacrylat-Acrylamid-Copolymere, Acrylsäureester-Methacrylsäureester-Copolymere, Ethylacrylat-Acrylsäure-Methacrylsäure-Copolymere, N-Methylolmethacrylamid, Maleinsäureanhydrid-Methylvinylether-Copolymere, Polyether-Polyol-Copolymere sowie Butadien-Styrol-Copolymere.

Weitere geeignete Verdicker sind Derivate organischer Säuren sowie deren Alkoxid-Addukte, beispielsweise Arylpolyglykolether, carboxylierte Nonylphenolethoxylatderivate, Natriumalginat, Diglycerinmonoisostearat, Nichtionogene Ethylenoxid-Addukte, Kokosfettsäurediethanolamid, Isododecenylbernsteinsäureanhydrid sowie Galactomannan.

Verdicker aus den genannten Substanzklassen sind kommerziell erhältlich und werden beispielsweise unter den Handelsnamen Acusol®-820 (Methacrylsäure(stearylalkohol-20-EO)-ester-Acrylsäure-Copolymer, 30%ig in Wasser, Rohm & Haas), Dapral®-GT-282-S (Alkylpolyglykolether, Akzo), Deuterol®-Polymer-11 (Dicarbonsäure-Copolymer, Schöner GmbH), Deuteron®-XG (anionisches Heteropolysaccharid auf Basis von β-D-Glucose, D-Manose, D-Glucuronsäure, Schöner GmbH), Deuteron®-XN (nichtionogenes Polysaccharid, Schöner GmbH), Dicrylan®-Verdicker-O (Ethylenoxid-Addukt, 50%ig in Wasser/Isopropanol, Pfersse Chemie), EMA®-81 und EMA®-91 (Ethylen-Maleinsäureanhydrid-Copolymer, Monsanto), Verdicker-QR-1001 (Polyurethan-Emulsion, 19-21%ig in Wasser/Diglykolether, Rohm & Haas), Mirox®-AM (anionische Acrylsäure-Acrylsäureester-Copolymer-Dispersion, 25%ig in Wasser, Stockhausen), SER-AD-FX-1100 (hydrophobes Urethanpolymer, Servo Delden), Shellflo®-S (hochmolekulares Polysaccharid, mit Formaldehyd stabilisiert, Shell), Shellflo®-XA (Xanthan-Biopolymer, mit Formaldehyd stabilisiert, Shell), Kelzan, Keltrol T (Kelco) angeboten.

In einer weiteren bevorzugten Ausführungsform enthält das Mittel gegebenenfalls ein oder mehrere Enzyme. Es können prinzipiell alle den Wasch- und Textilpflegeprozeß unterstützenden Enzyme eingesetzt werden. Bevorzugt ist jedoch der Einsatz von Cellulasen. Diese unterstützen nicht nur die Verringerung der Pillbildung und Flusenreduktion, sondern lassen sich ebenso gut in Flüssigformulierungen einarbeiten. Sie sind insbesondere deshalb bevorzugt, da sie auch bei niedrigen Temperaturen nicht aus den erfindungsgemäßen Formulierungen ausflocken und eine ausgesprochene Kältestabilität aufweisen. Sie werden vorzugsweise in Mengen von etwa 0,1 bis 5 Gew.-%, vorzugsweise 0,12 bis etwa 2 Gew.-%, jeweils bezogen auf das gesamte Mittel, eingearbeitet.

Die Enzyme können als Formkörper an Trägerstoffe adsorbiert oder gecoated eingebettet sein, um sie gegen vorzeitige Zersetzung zu schützen.

In einer besonders bevorzugten Ausführungsform enthält das Mittel Cellulasen.

Die Mittel können gegebenenfalls Bleichmittel enthalten. Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate beziehungsweise Perschwefelsäure. Brauchbar ist auch das Harnstoffperoxohydrat Percarbamid, das durch die Formel H₂N-CO-NH₂-H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphtoesäure und Magnesium-monoperphthalat, die aliphatischen oder substituiert aliphatischen Peroxysäuren, wie Peroxylaurinsäure, Peroxystearinsäure, ε-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure) können eingesetzt werden.

Die Bleichmittel können gecoated sein, um sie gegen vorzeitige Zersetzung zu schützen.

Farbstoffe können gegebenenfalls im erfindungsgemäßen Mittel als Ästhetikhilfsmittel eingesetzt werden.

Weiterhin kann das erfindungsgemäße Mittel gegebenenfalls einen oder mehrere antimikrobielle Wirkstoffe bzw. Konservierungsmittel in einer Menge von üblicherweise 0,0001 bis 3 Gew.-%, vorzugsweise 0,0001 bis 2 Gew.-%, insbesondere 0,0002 bis 1 Gew.-%, besonders bevorzugt 0,0002 bis 0,2 Gew.-%, äußerst bevorzugt 0,0003 bis 0,1 Gew.-%, enthalten.

Antimikrobielle Wirkstoffe bzw. Konservierungsmittel unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung, die beispielsweise von K. *H. Wallhäußer* in "Praxis der Sterilisation, Desinfektion - Konservierung : Keimidentifizierung - Betriebshygiene" (5. Aufl. - Stuttgart; New York : Thieme, 1995) wiedergegeben wird, wobei alle dort beschriebenen Substanzen mit antimikrobieller Wirkung eingesetzt werden können. Geeignete antimikrobielle Wirkstoffe sind vorzugsweise ausgewählt aus den Gruppen der Alkohole, Amine, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, Iodo-2-propyl-butyl-carbamat, Iod, Iodophore, Peroxoverbindungen, Halogenverbindungen sowie beliebigen Gemischen der voranstehenden.

Der antimikrobielle Wirkstoff kann dabei ausgewählt sein aus Ethanol, n-Propanol, i-Propanol, 1,3-Butandiol, Phenoxyethanol, 1,2-Propylenglykol, Glycerin, Undecylensäure, Benzoesäure, Salicylsäure, Dihydracetsäure, o-Phenylphenol, N-Methylmorpholin-acetonitril (MMA), 2-Benzyl-4-chlorphenol, 2,2'-Methylen-bis-(6-brom-4-chlorphenol), 4,4'-Dichlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-harnstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschl. den Bi- und Polyguanidinen, wie beispielsweise 1,6-Bis-(2-ethylhexyl-biguanido-hexan)-dihydrochlorid, 1,6-Di-(N₁,N₁'-phenyldiguanido-N₅,N₅')-hexan-tetrahydochlorid, 1,6-Di-(N₁,N₁'-phenyl-N₁,N₁-methyldiguanido-N₅,N₅')-hexan-dihydrochlorid, 1,6-Di-(N₁,N₁'-o-chlorophenyldiguanido- N₅,N₅')-hexan-dihydrochlorid, 1,6-Di-(N₁,N₁'-2,6-dichlorophenyldiguanido-N₅,N₅')hexan-dihydrochlorid, 1,6-Di-[N₁,N₁'-beta-(p-methoxyphenyl) diguanido-N₅,N₅']-hexane-dihydrochlorid, 1,6-Di-(N₁,N₁'-alpha-methyl-.beta.-phenyldiguanido-N₅,N₅')-hexan-dihydrochlorid, 1,6-Di-(N₁,N₁'-p-nitrophenyldiguanido-N₅,N₅')hexan-dihydrochlorid, omega:omega-Di-( N₁,N₁'-phenyldiguanido-N₅,N₅')-di-n-propyl-ether-dihydrochlorid, omega:omega'-Di-(N₁,N₁'-p-chlorophenyldiguanido-N₅,N₅')-di-n-propyl-ether-tetrahydrochlorid, 1,6-Di-(N₁,N₁'-2,4- dichlorophenyldiguanido-N₅,N₅')hexan-tetrahydrochlorid, 1,6-Di-(N₁,N₁'-p-methylphenyldiguanido- N₅,N₅')hexan-dihydrochlorid, 1,6-Di-(N₁,N₁'-2,4,5-trichlorophenyldiguanido-N₅,N₅')hexan-tetrahydrochlorid, 1,6-Di-[N₁,N₁'-alpha-(p-chlorophenyl) ethyldiguanido-N₅,N₅'] hexan-dihydrochlorid, omega:omega-Di-(N₁,N₁'-p-chlorophenyldiguanido-N₅,N₅')m-xylene-dihydrochlorid, 1,12-Di-(N₁,N₁'-p-chlorophenyldiguanido-N₅,N₅') dodecan-dihydrochlorid, 1,10-Di-(N₁,N₁'-phenyldiguanido- N₅,N₅')-decan-tetrahydrochlorid, 1,12-Di-(N₁,N₁'-phenyldiguanido- N₅,N₅') dodecan-tetrahydrochlorid, 1,6-Di-(N₁,N₁'-o-chlorophenyldiguanido- N₅,N₅') hexan-dihydrochlorid, 1,6-Di-(N₁,N₁'-o-chlorophenyldiguanido-N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbiguanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylen bis (o-tolylbiguanid), N-Butyl-trimethyle- bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlor-meta-xylol, sowie natürliche antimikrobielle Wirkstoffe pflanzlicher Herkunft (z.B. aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/ oder mindestens ein natürlicher antimikrobieller Wirkstoff tierischer Herkunft aus der Gruppe, umfassend Enzyme wie Eiweiß aus Milch, Lysozym und Lactoperoxidase, und/ oder mindestens eine antimikrobiell wirkende oberflächenaktive quaternäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden. Vorzugsweise finden Glycin, Glycinderivate, Formaldehyd, Verbindungen, die leicht Formaldehyd abspalten, Glutardialdehyd, Ameisensäure und Peroxide Verwendung.

Bei Einsatz des erfindungsgemäßen Konditioniermittels beispielsweise als Tränkflüssigkeit für Konditioniersubstrate eignet sich insbesondere Glutardialdehyd.

Die als antimikrobielle Wirkstoffe geeigneten quaternären Ammoniumverbindungen (QAV) weisen die allgemeine Formel (R¹)(R²)(R³)(R⁴) N⁺ X⁻ auf, in der R¹ bis R⁴ gleiche oder verschiedene C₁-C₂₂-Alkylreste, C₇-C₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid. unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielsweise Benzalkoniumchlorid (N-Alkyl-N,N-dimethyl-benzyl-ammoniumchlorid, CAS No. 8001-54-5), Benzalkon B (*m*,*p*-Dichlorbenzyl-dimethyl-C12-alkyl-ammoniumchlorid, CAS No. 58390-78-6), Benzoxoniumchlorid (Benzyl-dodecyl-bis-(2-hydroxyethyl)-ammonium-chlorid), Cetrimoniumbromid (N-Hexadecyl-N,N-trimethyl-ammoniumbromid, CAS No. 57-09-0), Benzetoniumchlorid (N,N-Dimethyl-N-[2-[2-[p-(1,1,3,3-tetramethylbutyl)-pheno-xy]ethoxy]ethyl]-benzylammoniumchlorid, CAS No. 121-54-0), Dialkyldimethylammonium-chloride wie Di-n-decyl-dimethyl-ammoniumchlorid (CAS No. 7173-51-5-5), Didecyldi-methylammoniumbromid (CAS No. 2390-68-3), Dioctyl-dimethyl-ammoniumchloric, 1-Cetylpyridiniumchlorid (CAS No. 123-03-5) und Thiazoliniodid (CAS No. 15764-48-1) sowie deren Mischungen. Besonders bevorzugte QAV sind die Benzalkoniumchloride mit C₈-C₁₈-Alkylresten, insbesondere C₁₂-C₁₄-Aklyl-benzyl-dimethyl-ammoniumchlorid.

Benzalkoniumhalogenide und/ oder substituierte Benzalkoniumhalogenide sind beispielsweise kommerziell erhältlich als Barquat^{®} ex Lonza, Marquat^{®} ex Mason, Variquat^{®} ex Witco/Sherex und Hyamine^{®} ex Lonza, sowie Bardac^{®} ex Lonza. Weitere kommerziell erhältliche antimikrobielle Wirkstoffe sind N-(3-Chlorallyl)-hexaminiumchlorid wie Dowicide^{®} und Dowicil^{®} ex Dow, Benzethoniumchlorid wie Hyamine^{®} 1622 ex Rohm & Haas, Methylbenzethoniumchlorid wie Hyamine^{®} 10X ex Rohm & Haas, Cetylpyridiniumchlorid wie Cepacolchlorid ex Merrell Labs.

Die Mittel können weiterhin gegebenenfalls UV-Absorber enthalten, die auf die behandelten Textilien aufziehen und die Lichtbeständigkeit der Fasern und/oder die Lichtbeständigkeit des sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, wie beispielsweise das wasserlösliche Benzolsulfonsäure-3-(2H-benzotriazol-2-yl)-4-hydroxy-5-(methylpropyl)-mononatriumsalz (Cibafast^{®} H), in 3-Stellung Phenylsubstituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb^{®} FD oder Tinosorb^{®} FR ex Ciba erhältlich sind. Als UV-B-Absorber sind zu nennen 3-Benzylidencampher bzw. 3-Benzylidennorcampher und dessen Derivate, z.B. 3-(4-Methylbenzyliden)campher, wie in der EP 0 693 471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie z.B. 2,4,6-Trianilino-(p-carbo-2'-ethyl-1'-hexyloxy)-1,3,5-triazin und Octyl Triazon, wie in der EP 0 818 450 A1 beschrieben oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)decan-Derivate, wie in der EP 0 694 521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie z.B. 4-(2-Oxo-3-bornylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 197 12 033 A1 (BASF). Die UV-A und UV-B-Filter können selbstverständlich auch in Mischungen eingesetzt werden.

Die UV-Absorber werden üblicherweise in Mengen von 0,01 Gew.-% bis 5 Gew.-%, vorzugsweise von 0,03 Gew.-% bis 1 Gew.-%, eingesetzt.

In einer bevorzugten Ausführungsform enthalten die erfindungsgemäßen Mittel Geruchsabsorber, vorzugsweise Zink-Ricinoleat oder Cyclodextrine.

Zweiter Gegenstand der Erfindung ist ein Verfahren zur Herstellung eines erfindungsgemäßen Konditioniermittels. Wie bereits beschreiben, kann das Verfahren in prinzipieller Anlehnung an dem Fachmann bekannte Verfahren ausgeführt werden.

Als besonders vorteilhaft für die Stabilität, Kältestabilität und Frostresistenz des erfindungsgemäß herstellbaren Konditioniermittels hat es sich jedoch erwiesen, die Weichmacherverbindung in die Reaktionsmischung einzudüsen, gegebenenfalls muß die Weichmacherverbindung dazu vorher aufgeschmolzen werden.

In einer bevorzugten Ausführungsform zeichnet sich das erfindungsgemäße Verfahren dadurch aus, daß die Weichmacherkomponente bei der Zugabe in flüssiger Form vorliegt, insbesondere geschmolzen, und in den Reaktionsansatz eingedüst wird. Vorteilhafterweise lassen sich die erfindungsgemäßen Esterquats der Formeln (a) und (b) problemlos bei der Eindüsung handhaben und sind bei dem Verfahren sehr gut in die Reaktionsmischung einarbeitbar.

Das Eindüsen der Weichmacherverbindung läßt sich beispielsweise dadurch realisieren, daß der Reaktionsmischung, die sich beispielsweise in einem Rührkessel befindet, die Weichmacherverbindung über ein Rohrsystem zugeführt wird, wobei die einzelnen Rohre, beispielsweise 5-10, mit einer Vielzahl von Löchern versehen sind, die beispielsweise Durchmesser unter 1 cm aufweisen.

In einer weiteren bevorzugten Ausführungsform zeichnet sich das erfindungsgemäße Verfahren dadurch aus, daß die Zugabe der Weichmacherkomponente, des Cryoprotektors und/oder der anorganischen Salze zum Reaktionsansatz portionsweise erfolgt, insbesondere in 2, 3 oder 4 Portionen. Dabei versteht man unter dem Begriff der portionsweisen Zugabe, daß die Gesamtmenge der zuzugebenden Komponente nicht in einer einzigen Portion zugegeben wird. Liegen beispielsweise 600 g zuzugebender Komponente vor, so teilt man diese z. B. in drei Portionen auf, die beispielsweise annähernd gleich groß sind, etwa in drei Portionen a 200 g. Um bei diesem Beispiel zu bleiben, gibt man also zuerst 200 g der Komponente zum Reaktionsansatz, läßt beispielsweise 5 Minuten verstreichen und gibt die nächste Portion a 200 g zu, dies setzt man fort, bis alle Portionen zugegeben sind.

Es wurde überraschenderweise von der Anmelderin gefunden, daß die portionsweise Zugabe dieser Komponenten zu einer weiteren Verbesserung der Frostresistenz und Kältestabilität der erfindungsgemäß herzustellenden Mittel führt. Die Anmelderin führt dies, ohne sich an eine bestimmte Theorie zu binden, darauf zurück, daß die portionsweise, sukzessive Zugabe eine verbesserte Homogenität des herzustellenden Mittels bei stabiler Viskosität bewirkt und dadurch die Frostresistenz unterstützt. Es konnte beobachtet werden, daß die Viskosität bei einer portionsweisen Zugabe der genannten Komponeten sehr viel weniger stark ansteigt als bei einer einmaligen Zugabe. Dies gilt besonders dann, wenn bestimmte Mengen an zuzugebender Weichmacherkomponente überschritten werden. Werden mehr als 8 Gew.-% an Weichmacherkomponente zugegeben, so soll die Zugabe bevorzugt portionsweise - vorzugsweise in 2 Portionen a ⅓ und a ²/₃ - erfolgen, unterbrochen von ca. 5 minütigem Rühren zwischen den einzelnen Portionszugaben.

In einer weiteren bevorzugten Ausführungsform zeichnet sich das erfindungsgemäße Verfahren dadurch aus, daß die Zugabe des Cryoprotektors in den Reaktionsansatz vor der Parfumölzugabe erfolgt.

Es konnte von der Anmelderin überraschend gefunden werden, daß die Zugabe des Cryoprotektors vor der Parfumölzugabe zu einer weiteren Verbesserung der Kätestabilität führt.

In einer weiteren bevorzugten Ausführungsform zeichnet sich das erfindungsgemäße Verfahren dadurch aus, daß die Zugabe des Cryoprotektors in den Reaktionsansatz portionsweise erfolgt. Dabei versteht man unter dem Begriff der portionsweisen Zugabe das zuvor gesagte.

Es konnte von der Anmelderin überraschend gefunden werden, daß die portionsweise Zugabe des Cryoprotektors zu einer weiteren Verbesserung der Kätestabilität führt.

In einer weiteren bevorzugten Ausführungsform zeichnet sich das erfindungsgemäße Verfahren dadurch aus, daß sowohl die Zugabe des Cryoprotektors in den Reaktionsansatz als auch die Zugabe der Weichmacherkomponente unter schnellem Rühren, beispielsweise mit einer Winkelgeschwindigkeit von 6 m/s oder größer, erfolgt.

Im Zusammenhang mit der Rührgeschwindigkeit wurde von der Anmelderin überraschend gefunden, daß eine Erhöhung der Rührgeschwindigkeit zu einer weiteren Verbesserung der Frostresistenz des erfindungsgemäß herzustellenden Mittels führt. Die Anmelderin interpretiert den Einfluß der Rührgeschwindigkeit, ohne sich an eine bestimmte Theorie zu binden, dahingehend, daß bei einer Rührgeschwindigkeitserhöhung eine verbesserte Homogenität des herzustellenden Mittels bewirkt wird und dadurch dessen Stabilität, Kältestabilität und Frostresistenz unterstützt wird. Wird der Reaktionsansatz zu langsam gerührt oder durchmischt, so treten bei der Zugabe von Cryoprotektor und Weichmacherkomponente sichtbare Inhomogenitäten in der Reaktionsmischung auf, beispielsweise kommt es dann zu einer Klumpenbildung.

In einer besonderen Ausführungsform zeichnet sich das erfindungsgemäße Verfahren dadurch aus, daß es sich um einen kontinuierlichen Prozess handelt. Selbstverständlich kann das Verfahren auch batchweise geführt werden.

Dritter Gegenstand der Erfindung ist die Verwendung des erfindungsgemäßen Mittels zur Konditionierung von textilen Geweben, insbesondere in einem Nachspülgang in einem

Waschverfahren. Unter dem Begriff der Konditionierung von textilen Geweben ist im Sinne dieser Erfindung die avivierende Behandlung von Textilien, Stoffen und Geweben zu verstehen. Durch die Konditionierung werden den Textilien positive Eigenschaften verliehen, wie beispielsweise ein verbesserter Weichgriff, eine erhöhte Glanz- und Farbbrillanz, Verringerung des Knitterverhaltens und der statischen Aufladung.

Vierter Gegenstand der Erfindung ist die Verwendung des erfindungsgemäßen Mittels zur Konditionierung von keratinen Fasern, insbesondere also im Sinne einer kosmetischen Anwendung, beispielsweise zur kosmetischen Haarpflege. Durch die Konditionierung werden den keratinen Fasern, beispielsweise menschlichem Haar, positive Eigenschaften verliehen, wie beispielsweise eine verbesserte Kämmbarkeit, eine erhöhte Glanz- und Farbbrillanz, Verringerung der Fasersprödigkeit und der statischen Aufladung der Faser.
In einer bevorzugten Ausführungsform gelangt dabei ein Sprühspender zur Anwendung.

Ein weiterer Gegenstand der Erfindung ist ein Erzeugnis, enthaltend ein erfindungsgemäßes Konditioniermittel und einen Sprühspender.

Bevorzugt ist der Sprühspender ein manuell aktivierbarer Sprühspender, insbesondere ausgewählt aus der Gruppe, umfassend Aerosolsprühspender, selbst Druck aufbauende Sprühspender, Pumpsprühspender und Triggersprühspender, insbesondere Pumpsprühspender und Triggersprühspender mit einem Behälter aus transparentem Polyethylen oder Polyethylenterephthalat.

Solche und ähnliche Sprühspender oder damit verwandte Applikationsvorrichtungen sind handelsüblich und sämtliche handelsüblichen Sprühspender oder verwandte Applikationsvorrichtungen kommen zur erfindungsgemäßen Applikation in Betracht.

Demgemäß ist ein weiterer Gegenstand der Erfindung ein Verfahren zur Konditionierung textiler Gewebe oder keratiner Fasern, bei dem eine wirksame Menge eines erfindungsgemäßen Mittels, vorzugsweise unter Verwendung eines eben beschriebenen Erzeugnisses, auf das zu behandelnde Substart vorzugsweise durch Sprühen aufgebracht wird. Unter einer wirksamen Menge wird dabei ein Menge verstanden, die eine wunschgemäße Konditionierungsintensität ermöglicht. Diese Menge ist eine individuelle, die von vielen Faktoren wie z. B. Substrattyp und -zustand, gewünschtes bzw. zu erzielendes Ergebnis abhängt.

In einer bevorzugten Ausführungsform des eben genannten Verfahrens wird das erfindungsgemäße Mittel, insbesondere unter Verwendung eines erfindungsgemäßen Erzeugnisses, auf das zu konditionierende Substrat, insbesondere aus einer Entfernung von 10 bis 100 cm, vorzugsweise 20 bis 50 cm, besonders bevorzugt 25 bis 40 cm, äußerst bevorzugt etwa 30 cm, gesprüht.

Der besondere Vorteil der vorgenannten Gegenstände, die sich im weitesten Sinne auf die Verwendung eines Sprühspenders beziehen liegt darin, daß die Sprühfähigkeit des zu versprühenden Konditioniermittels auch bei tiefen Temperaturen im Sinne der Erfindung gewahrt bleibt.

### Beispiele

### Herstellung eines efindungsgemäßen Konditioniermittels am Beispiel eines Weichspülers; Beispiel 1

1301,5 g Wasser werden mit 70°C bei leichter Rührung vorgelegt. 117,7 g Rewoquat WE 18 (Di-(Talgcarboxyethyl)-hydroxyethyl-methylammoniummethosulfat; 90 Gew.-% in Isopropanol; Bezugsquelle: Degussa), vortemperiert auf 50°C, werden zugegeben. Nach 10 Minuten leichter Rührung gibt man weitere 235,3 g Rewoquat WE 18, vortemperiert auf 50°C, unter nun starker Rührung zu. Anschließend wird, unter Beibehaltung der starken Rührung 12 g MgCl_{*}6H₂0, gelöst in 24 g Wasser zugegeben, und es wird wieder eine leichte Rührung eingestellt. Nach 5 minütiger Rührzeit wird auf 30°C abgekühlt, dabei werden weitere 687 g Wasser zugegeben. Dann werden 3 mal je 200 g Dipropylenglykol zugegeben, wobei zwischen den Portionszugaben je 5 Minuten bei leichtem Rühren gewartet wird.
Abschließend werden 6 g Eumulgin RO 40 (Rizinusöl-ethoxyliert, 40 EO; Bezugsquelle: Cognis Deutschland GmbH), vorgemischt mit 7,5 g eines hydrophoben Parfumöls zugegeben und 15 Minuten weitergerührt.

Bezogen auf den Gesamtansatz ( 2991 g ) bedeutet dies kurz zusammengefaßt: Di-(Talgcarboxyethyl)-hydroxyethyl-methylammonium-

| | |
|---|---|
| methosulfat | 10,6 Gew.-% (Zugabe in 2 Portionen) |
| Dipropylenglykol | 20 Gew.-% (Zugabe in 3 Portionen) |
| M₉Cl_{2*}6H₂0 | 0,4 Gew.-% |
| Eumulgin RO 40 | 0,2 Gew.-% |
| Parfumöl, hydrophob | 0,25 Gew.-% |
| Isopropanol (ex Rewoquat WE 18) | 1,2 Gew.-% |
| Wasser | ad 100 |

Das Produkt zeigt bei Raumtemperatur (20°C) eine ausgezeichnete Verteilbarkeit.

### Lagertests:

Lagerung bei Raumtemperatur (20°C)
   η (20°C/ nach 1 Tag Lagerung) = 25mPas; ausgezeichnete Verteilbarkeit
   η (20°C/ nach 2 Tagen Lagerung) = 25mPas; ausgezeichnete Verteilbarkeit
   η (20°C/ nach 5 Tagen Lagerung) = 25mPas; ausgezeichnete Verteilbarkeit
   η (20°C/ nach 7 Tagen Lagerung) = 30mPas; ausgezeichnete Verteilbarkeit
   η (20°C/ nach 60 Tagen Lagerung) =25mPas; sehr gute Verteilbarkeit

Lagerung bei Frost
   η (4 Tage Lagerung bei -13°C, danach 4 Tage Lagerung bei 20°C) =160mPas; sehr gute Verteilbarkeit
   η (5 Tage Lagerung bei -16°C, danach 4 Tage Lagerung bei 20°C) =250mPas; gute Verteilbarkeit
   Eine Phasentrennung, Gelbildung, Ausfällung oder ähnliches wird nach keinem der Lagertests beobachtet. Der trübe Weichspüler ist bei einer Temperatur von -16°C nicht erstarrt.

Die Verteilbarkeit des zu prüfenden, trüben Weichspülers wird nach folgender Prozedur beurteilt:

Ein 150 ml Becherglas wird mit 75 ml kaltem Leitungswasser befüllt. 25 ml des zu prüfenden Weichspülers werden in das Wasser hineingegossen und die Verteilbarkeit optisch begutachtet.

Die Viskosität η wurde mit einem Brookfield-Viskosimeter Typ RV bei 20 U/min und 20°C, Spindel 1, gemessen.

### Erfindungsgemäßes Konditioniermittel am Beispiel eines Weichspülers; Beispiel 2

### Verfahren analog Beispiel 1

### Für diesen Ansatz gilt in Analogie zu Beispiel1:

Di-(Talgcarboxyethyl)-hydroxyethyl-methylammonium-

| | |
|---|---|
| methosulfat | 10 Gew.-% (Zugabe in 2 Portionen) |
| Dipropylenglykol | 20 Gew.-% (Zugabe in 3 Portionen) |
| MgCl₂.6H₂0 | 0,4 Gew.-% |
| Eumulgin RO 40 | 0,125 Gew.-% |
| Parfumöl, hydrophob | 0,5 Gew.-% |
| Isopropanol (ex Rewoquat WE 18) | 1,1 Gew.-% |
| Wasser | ad 100 |

Der trübe Weichspüler zeigt bei Raumtemperatur (20°C) eine ausgezeichnete Verteilbarkeit.

### Lagertests:

Lagerung bei Raumtemperatur (20°C)
   η (20°C/ nach 7 Tagen Lagerung) = 25mPas; ausgezeichnete Verteilbarkeit
Lagerung bei Frost
   η (4 Tage Lagerung bei -16°C, danach 4 Tage Lagerung bei 20°C) =275mPas; gute Verteilbarkeit
Eine Phasentrennung, Gelbildung, Ausfällung oder ähnliches wird nach keinem der beiden Lagertests beobachtet. Das Mittel ist bei einer Temperatur von -16°C nicht erstarrt.

## Patentansprüche

1. Trübes, frostresistentes, flüssiges, wässriges Konditioniermittel, enthaltend einen oder mehrere mehrwertige Alkohole und/oder Glycolether als Cryoprotektoren, insbesondere in einer Menge größer 5 Gew.-% bezogen auf das gesamte Mittel, sowie eine Weichmacherkomponente, ausgewählt aus den nachfolgenden Formeln (a): 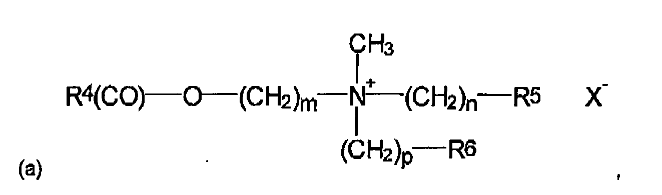 hierbei steht R⁴ für einen aliphatischen Alkylrest mit 12 bis 22 Kohlenstoffatomen mit 0, 1, 2 oder 3 Doppelbindungen; R⁵ steht für H, OH oder insbesondere O(CO)R⁷, R⁶ steht unabhängig von R⁵ für H, OH oder O(CO)R⁹, wobei R⁷ und R⁸ unabhängig voneinander jeweils für einen aliphatischen Alkylrest mit 12 bis 22 Kohlenstoffatomen mit 0, 1, 2 oder 3 Doppelbindungen steht, m, n und p können jeweils unabhängig voneinander den Wert 1, 2 oder 3 haben, X⁻ ist ein passendes Anion, vorzugsweise ein Halogenid-, Methosulfat-, Methophosphat- oder Phosphation sowie Mischungen aus diesen,
oder der Formel (b) 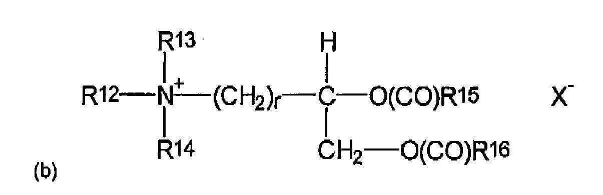 wobei R¹², R¹³ und R¹⁴ unabhängig voneinander für eine C₁₋₄-Alkyl-, Alkenyl- oder Hydroxyalkylgruppe steht, R¹⁵ und R¹⁶ jeweils unabhängig ausgewählt eine C₈₋₂₈-Alkylgruppe mit 0, 1, 2 oder 3 Doppelbindungen darstellt und r eine Zahl zwischen 0 und 5 ist, X⁻ ist ein passendes Anion, vorzugsweise ein Halogenid-, Methosulfat-, Methophosphat- oder Phosphation sowie Mischungen aus diesen
sowie wenigstens ein Alkoholethoxylat mit wenigstens 20 EO-Einheiten.

2. Konditioniermittel nach Anspruch 1, **dadurch gekennzeichnet, daß** es Weichmacherkomponenten in Mengen bis zu 25 Gew.-%, vorzugsweise von 0,1 bis 24 Gew.-%, besonders bevorzugt von 2 bis 22 Gew.-%, äußerst bevorzugt von 3 bis 20 Gew.-% und insbesondere von 5 bis 18 Gew.-%, jeweils bezogen auf das gesamte Mittel, enthält.

3. Konditloniermittel nach den Ansprüchen 1-2, **dadurch gekennzeichnet, daß** die quartären Verbindungen gemäß Formeln (a) oder (b) ungesättigte Alkylketten aufweisen, wobei die korrespondierenden Fettsäuren eine Jodzahl zwischen 5 und 80, vorzugsweise zwischen 10 und 60 und insbesondere zwischen 30 und 50 aufweisen, sowie vorzugsweise ein cis/trans-Isomerenverhältnis (in Gew.-%) von größer als 30 : 70, vorzugsweise größer als 50: 50 und insbesondere größer als 60 : 40 haben.

4. Konditioniermittel nach Anspruch 3, **dadurch gekennzeichnet, daß** es sich bei den korrespondierenden Fettsäuren um teilgehärtete Talgfettsäure handelt.

5. Konditioniermittel nach den Ansprüchen 1-4, **dadurch gekennzeichnet, daß** die Erstarrungstemperatur des Mittels maximal -1°C, bevorzugt maximal -5°C, besonders maximal -10°C, insbesondere maximal -15°C beträgt.

6. Konditioniermittel nach den Ansprüchen 1-5, **dadurch gekennzeichnet, daß** es Dipropylenglykol als Cryoprotektor enthält, insbesondere in einer Menge größer 15 Gew.-%, bezogen auf das gesamte Mittel.

7. Konditioniermittel nach den Ansprüchen 1-6, **dadurch gekennzeichnet, daß** das Alkoholethoxylat wenigstens 30 EO-Einheiten enthält.

8. Konditioniermittel nach den Ansprüchen 1-7, **dadurch gekennzeichnet, daß** Mittel einen Gesamtgehalt an Alkoholethoxylat, jeweils bezogen auf das Mittel, von unter 10 Gew.-%, vorzugsweise unter 5 Gew.-%, bevorzugt unter 1 Gew.-%, insbesondere aber zwischen 0,1 bis 0,6 Gew.-% , weiter bevorzugt zwischen 0,15 bis 0,5 Gew.-%, jedoch besonders bevorzugt zwischen 0,2 bis 0,4 Gew.-% aufweist.

9. Konditioniermittel nach den Ansprüchen 1-8, **dadurch gekennzeichnet, daß** es sich bei dem Alkoholethoxylat um ein hochethoxyliertes Rizinusöl mit wenigstens 20 EO insbesondere wenigstens 30 EO-Einheiten handelt.

10. Konditioniermittel nach den Ansprüchen 1-9, **dadurch gekennzeichnet, daß** es Magnesiumchlorid, vorzugsweise Magnesiumchloridhexahydrat enthält, insbesondere in Mengen von 0,005 bis 8 Gew.-% bezogen auf das Mittel.

11. Konditioniermittel nach den Ansprüchen 1-10, **dadurch gekennzeichnet, daß** das Mittel zusätzlich eine zwitterionische Verbindung nachstehender Formel vorzugsweise in Mengen bis zu 10 Gew.-% enthält: 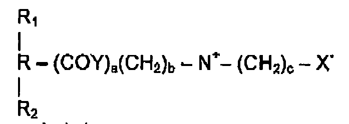 wobei das R für eine C₆₋₂₆-Alkyl- oder Alkenylgruppe steht; R₁ und R₂ sind jeweils, unabhängig voneinander, C₁₋₄ Alkyl-Gruppen; a steht für die Zahl 0 oder 1, b und c sind jeweils, unabhängig voneinander, aus ganzen Zahlen von 1 bis 4 ausgewählt; Y ist Sauerstoff oder Stickstoff; X ist ein kompatibles Anion.

12. Konditioniermittel nach den Ansprüchen 1-11, **dadurch gekennzeichnet, daß** das Mittel zusätzlich ein Alkylamidoalkylendimethylcarbonsäure-Betain mit der nachstehenden Formel enthält: 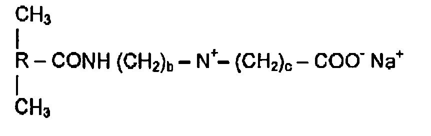 wobei b und c, unabhängig voneinander, ganze Zahlen von 1-4 sind, vorzugsweise b=2 oder 3 und c=2 oder 3 und wobei R für eine C₁₀₋₁₆-Alkylkette oder Mischungen davon steht.

13. Konditioniermittel nach den Ansprüchen 1-12, **dadurch gekennzeichnet, daß** das Mittel zusätzlich Fettsäuren enthält, vorzugsweise solche mit einer Iodzahl von 0 bis 25, vorzugsweise ausgewählt aus der folgenden Gruppe Laurin-, Tridecan-, Myristin-, Pentadecan-, Palmitin-, Margarin-, Stearin-, Nonadecan-, Arachin-, Behen-, Lignocerin-, Cerotin-, Melissinsäure, Palmitolein-, Öl-, Erucasäure, Linol-, Linolen-, Elaeostearin-, Arachidonsäure, wobei das Gewichtsverhältnis von Weichmacherkomponente zu Fettsäure vorzugsweise 25: 1 bis 5:1 beträgt.

14. Konditioniermittel nach den Ansprüchen 1-13, **dadurch gekennzeichnet, daß** ein Parfum, vorzugsweise ein hydrophobes Parfum, enthalten ist.

15. Konditioniermittel nach den Ansprüchen 1-14, **dadurch gekennzeichnet, daß** es als Dispersion vorliegt und eine mittlere Partikelgröße von kleiner 500 µm, besonders bevorzugt von kleiner 300 µm, äußerst bevorzugt von kleiner 200 µm hat, insbesondere liegt die mittlere Partikelgröße zwischen 0,005 µm und 180 µm.

16. Verfahren zur Herstellung eines Mittels nach einem der Ansprüche 1-15, **dadurch gekennzeichnet, daß** die Weichmacherkomponente bei der Zugabe in flüssiger Form vorliegt, insbesondere geschmolzen, und in den Reaktionsansatz eingedüst wird.

17. Verfahren nach Anspruch 16, **dadurch gekennzeichnet, daß** die Zugabe der Weichmacherkomponente und/oder des Cryoprotektors zum Reaktionsansatz portionsweise erfolgt.

18. Verfahren nach den Ansprüchen 16-17, **dadurch gekennzeichnet, daß** die Zugabe des Cryoprotectors vor der Parfumölzugabe erfolgt.

19. Verfahren nach den Ansprüchen 16-18, **dadurch gekennzeichnet, daß** die Zugabe der Textilweichmacherkomponente und des Cryoprotektors unter schnellem Rühren erfolgt.

20. Verfahren nach den Ansprüchen 16-19, **dadurch gekennzeichnet, daß** es sich um einen kontinuierlichen Prozess handelt.

21. Verwendung des Mittels nach einem der Ansprüche 1-15 zur Konditionierung von textilen Geweben, insbesondere in einem Nachspülgang in einem Waschverfahren.

22. Verwendung des Mittels nach einem der Ansprüche 1-15 zur Konditionierung keratiner Fasern, insbesondere unter Gebrauch eines Sprühspenders.

23. Verwendung eines Mittels nach einem der Ansprüche 1-15 als kosmetische Formulierung.

24. Erzeugnis, enthaltend ein Mittel nach einem der Ansprüche 1-15 und einen Sprühspender.

## Claims

1. A turbid, frost-resistant, liquid, aqueous conditioning agent containing one or more polyhydric alcohols and/or glycol ethers as cryoprotectants, in particular in an amount of greater than 5 wt.% relative to the entire agent, together with a softener component selected from the following formulae (a): 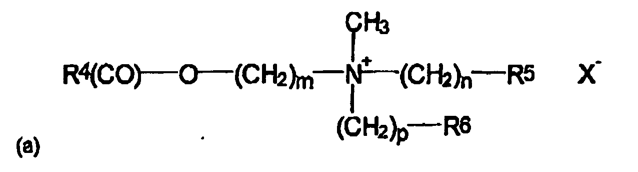 R⁴ here denotes an aliphatic alkyl residue with 12 to 22 carbon atoms with 0, 1, 2 or 3 double bonds; R⁵ denotes H, OH or in particular O(CO)R⁷, R⁶, independently of R⁵, denotes H, OH or O(CO)R⁸, wherein R⁷ and R⁸ mutually independently in each case denote an aliphatic alkyl residue with 12 to 22 carbon atoms with 0, 1, 2 or 3 double bonds, m, n and p may in each case mutually independently have the value 1, 2 or 3, X⁻ is an appropriate anion, preferably a halide, methosulfate, methophosphate or phosphate ion and mixtures of these,
or of the formula (b) 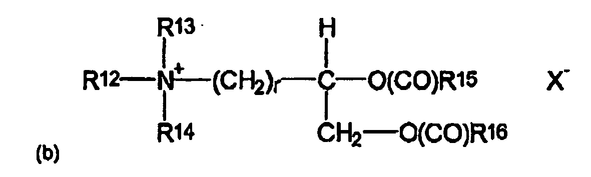 wherein R¹², R¹³ and R¹⁴ mutually independently denote a C₁₋₄ alkyl, alkenyl or hydroxyalkyl group, R¹⁵ and R¹⁶ in each case independently selected represent a C₈₋₂₈ alkyl group with 0, 1, 2 or 3 double bonds and r is a number between 0 and 5, X⁻ is an appropriate anion, preferably a halide, methosulfate, methophosphate or phosphate ion and mixtures of these
and at least one alcohol ethoxylate with at least 20 EO units.

2. A conditioning agent according to claim 1, **characterised in that** it contains softener components in amounts of up to 25 wt.%, preferably of 0.1 to 24 wt.%. particularly preferably of 2 to 22 wt.%, extremely preferably of 3 to 20 wt.% and in particular of 5 to 18 wt.%, in each case relative to the entire agent.

3. A conditioning agent according to claims 1-2, **characterised in that** the quaternary compounds according to formulae (a) or (b) comprise unsaturated alkyl chains, wherein the corresponding fatty acids have an iodine value of between 5 and 80, preferably between 10 and 60 and in particular between 30 and 50, preferably together with a cis/trans isomer ratio (in wt.%) of greater than 30:70, preferably greater than 50:50 and in particular greater than 60:40.

4. A conditioning agent according to claim 3, **characterised in that** the corresponding fatty acids comprise a partially hardened tallow fatty acid.

5. A conditioning agent according to claims 1-4, **characterised in that** the solidification temperature of the agent amounts to at most -1°C, preferably at most -5°C, particularly at most -10°C, in particular at most -15°C.

6. A conditioning agent according to claims 1-5, **characterised in that** it contains dipropylene glycol as cryoprotectant, in particular in an amount of greater than 15 wt.%, relative to the entire agent.

7. A conditioning agent according to claims 1-6, **characterised in that** the alcohol ethoxylate contains at least 30 EO units.

8. A conditioning agent according to claims 1-7, **characterised in that** agent has a total content of alcohol ethoxylate, in each case relative to the agent, of below 10 wt.%, preferably below 5 wt.%, preferably below 1 wt.%, but in particular between 0.1 and 0.6 wt.% , further preferably between 0.15 and 0.5 wt.%, but particularly preferably between 0.2 and 0.4 wt.%.

9. A conditioning agent according to claims 1-8, **characterised in that** the alcohol ethoxylate comprises a highly ethoxylated castor oil with at least 20 EO, in particular at least 30 EO units.

10. A conditioning agent according to claims 1-9, **characterised in that** it contains magnesium chloride, preferably magnesium chloride hexahydrate, in particular in amounts of 0.005 to 8 wt.% relative to the agent.

11. A conditioning agent according to claims 1-10, **characterised in that** the agent additionally contains a zwitterionic compound of the following formula preferably in amounts of up to 10 wt.%: 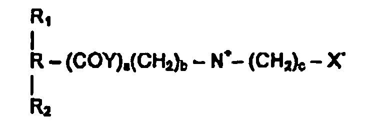 wherein R denotes a C₆₋₂₈ alkyl or alkenyl group; R₁ and R₂ are in each case, mutually independently, C₁₋₄ alkyl groups; a denotes the number 0 or 1, b and c are in each case, mutually independently, selected from integers from 1 to 4; Y is oxygen or nitrogen; X is a compatible anion.

12. A conditioning agent according to claims 1-11, **characterised in that** the agent additionally contains an alkylamidoalkylene dimethylcarboxylic acid betaine with the following formula: 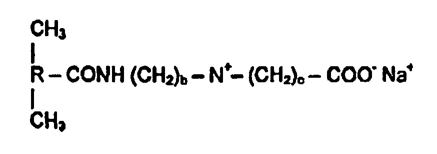 wherein b and c, mutually independently, are integers from 1-4, preferably b=2 or 3 and c=2 or 3 and wherein R denotes a C₁₀₋₁₈ alkyl chain or mixtures thereof.

13. A conditioning agent according to claims 1-12, **characterised in that** the agent additionally contains fatty acids, preferably those with an iodine value of 0 to 25, preferably selected from the following group lauric, tridecanoic, myristic, pentadecanoic, palmitic, margarinic, stearic, nonadecanoic, arachidic, behenic, lignoceric, cerotic, melissic acid, palmitoleic, oleic, erucic acid, linoleic, linolenic, elaeostearic, arachidonic acid, wherein the weight ratio of softener component to fatty acid preferably amounts to 25:1 to 5:1.

14. A conditioning agent according to claims 1-13, **characterised in that** it contains a perfume, preferably a hydrophobic perfume.

15. A conditioning agent according to claims 1-14, **characterised in that** it is in dispersion form and has an average particle size of less than 500 µm, particularly preferably of less than 300 µm, extremely preferably of less than 200 µm, the average particle size in particular being between 0.005 µm and 180 µm.

16. A method for producing an agent according to any one of claims 1-15, **characterised in that** the softener component is in liquid, in particular molten, form on addition and is injected into the reaction batch.

17. A method according to claim 16, **characterised in that** the softener component and/or the cryoprotectant is/are added to the reaction batch in portions.

18. A method according to claims 16-17, **characterised in that** the cryoprotectant is added before addition of the perfume oil.

19. A method according to claims 16-18, **characterised in that** the fabric softener component and the cryoprotectant are added with rapid stirring.

20. A method according to claims 16-19, **characterised in that** a continuous process is involved.

21. Use of the agent according to any one of claims 1-15 for conditioning textile fabrics, in particular in a post-rinsing cycle in a washing process.

22. Use of the agent according to any one of claims 1-15 for conditioning keratin fibres, in particular involving the use of a spray dispenser.

23. Use of an agent according to any one of claims 1-15 as a cosmetic formulation.

24. A product containing an agent according to any one of claims 1-15 and a spray dispenser.

## Revendications

1. Agent de conditionnement trouble, résistant au gel, liquide, aqueux, contenant un ou plusieurs alcools plurivalents et/ou éthers de glycol à titre de d'agents cryoprotecteurs, en particulier en une quantité supérieure à 5% en poids, par rapport à l'agent total, ainsi qu'un composant adoucisseur, choisi parmi les formules suivantes (a) : 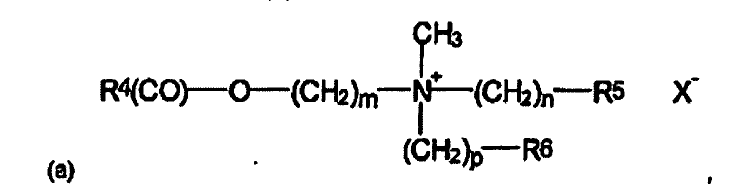 ici R⁴ représente un résidu alkyle aliphatique ayant de 12 à 22 atomes de carbone avec 0, 1, 2 ou 3 doubles liaisons ; R⁵ représente H, OH, ou en particulier O(CO)R⁷, R⁶ représente indépendamment de R⁵ H, OH, ou O(CO)R⁸ dans lesquels R⁷ et R⁸ représentent chacun indépendamment l'un de l'autre un résidu alkyle aliphatique ayant 12 à 22 atomes de carbone avec 0, 1, 2, ou 3 doubles liaisons, m, n et p peuvent avoir chacun indépendamment l'un de l'autre la valeur 1, 2 ou 3, X⁻ est un anion approprié, de préférence un ion halogénure, méthosulfate, méthophosphate ou phosphate ainsi que des mélanges de ceux-ci,
ou la formule (b) 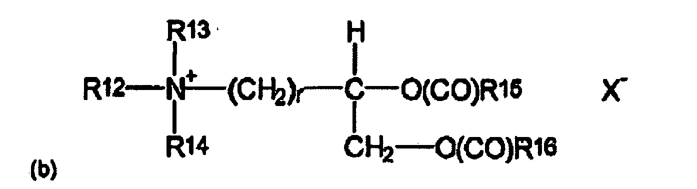 dans laquelle R¹², R¹³ et R¹⁴ représentent chacun indépendamment l'un de l'autre un groupe alkyle, alcényle, ou hydroxyalkyle en C₁ à C₄, R¹⁵ et R¹⁶, chacun indépendamment choisi, représentent un groupe alkyle en C₈ à C₂₈ avec 0, 1, 2 ou 3 doubles liaisons et r est un nombre compris entre 0 et 5, X⁻ est un anion approprié, de préférence un ion halogénure, méthosulfate, méthophosphate ou phosphate ainsi que des mélanges de ceux-ci,
ainsi qu'au moins un éthoxylate d'alcool ayant au moins 20 unités OE.

2. Agent de conditionnement selon la revendication 1, **caractérisé en ce qu'**il comprend des composants adoucisseurs en des quantités allant jusqu'à 25% en poids, de préférence de 0,1 à 24% en poids, de façon particulièrement préférée de 2 à 22% en poids, de façon tout particulièrement préférée de 3 à 20% en poids et en particulier de 5 à 18% en poids, à chaque fois par rapport à l'agent total.

3. Agent de conditionnement selon les revendications 1 à 2, **caractérisé en ce que** les composés quaternaires selon les formules (a) ou (b) présentent des chaînes alkyle insaturées, les acides gras correspondants présentant un indice d'iode compris entre 5 et 80, de préférence compris entre 10 et 60 et en particulier entre 30 et 50, ainsi que de préférence un rapport d'isomère *cis*/*trans* (% en poids) supérieur à 30:70, de préférence supérieur à 50:50 et en particulier supérieur à 60:40.

4. Agent de conditionnement selon la revendication 3, **caractérisé en ce que**, quant aux acides gras correspondants, il s'agit d'acide gras de suif partiellement durci.

5. Agent de conditionnement selon les revendications 1 à 4, **caractérisé en ce que** la température de solidification de l'agent est au maximum de -1 °C, de préférence au maximum de -5°C, particulièrement au maximum de -10°C, en particulier au maximum de -15°C.

6. Agent de conditionnement selon les revendications 1 à 5, **caractérisé en ce qu'**il contient, à titre d'agent cryoprotecteur le dipropyleneglycol, en particulier en une quantité supérieure à 15% en poids, par rapport à l'agent total.

7. Agent de conditionnement selon les revendications 1 à 6, **caractérisé en ce que** l'éthoxylate d'alcool contient au moins 30 unités OE.

8. Agent de conditionnement selon les revendications 1 à 7, **caractérisé en ce que** l'agent présente une teneur totale en éthoxylate d'alcool, à chaque fois par rapport à l'agent, de moins de 10% en poids, de préférence de moins de 5% en poids, préférentiellement de moins de 1% en poids, en particulier toutefois entre 0,1 à 0,6% en poids, très préférentiellement entre 0,15 à 0,5% en poids, toutefois de façon particulièrement préférée entre 0,2 à 0,4% en poids.

9. Agent de conditionnement selon les revendications 1 à 8, **caractérisé en ce que**, quant à l'éthoxylate d'alcool, il s'agit d'une huile de ricin hautement éthoxylée ayant au moins 20 unités OE, en particulier au moins 30 unités OE.

10. Agent de conditionnement selon les revendications 1 à 9, **caractérisé en ce qu'**il contient du chlorure de magnésium, de préférence du chlorure de magnésium hexahydraté, en particulier en des quantités de 0,005 à 8% en poids par rapport à l'agent.

11. Agent de conditionnement selon les revendications 1 à 10, **caractérisé en ce que** l'agent contient en outre un composé zwitterionique de la formule suivante de préférence en des quantités allant jusqu'à 10% en poids : 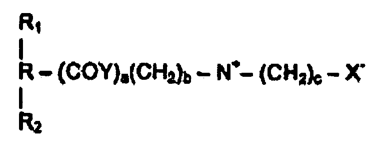 dans laquelle, R représente un groupe alkyle ou alcényle en C₆ à C₂₈ ; R₁ et R₂ sont chacun indépendamment l'un de l'autre des groupes alkyle en C₁ à C₄ ; a représente le nombre 0 ou 1, b et c sont chacun indépendamment l'un de l'autre choisis parmi les nombres entiers de 1 à 4 ; Y est un atome d'oxygène ou d'azote ; X est un anion compatible.

12. Agent de conditionnement selon les revendications 1 à 11, **caractérisé en ce que** l'agent contient en outre une bétaïne d'acide alkylamido-alkylène-diméthylcarboxylique ayant la formule suivante : 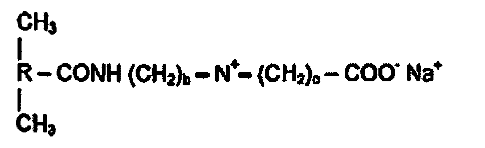 dans laquelle b et c indépendamment l'un de l'autre sont des nombres entiers de 1 à 4, de préférence b = 2 ou 3 et c = 2 ou 3 et R représente une chaîne alkyle en C₁₀ à C₁₈ ou des mélanges de celles-ci.

13. Agent de conditionnement selon les revendications 1 à 12, **caractérisé en ce que** l'agent contient en outre des acides gras, de préférence ceux ayant un indice d'iode de 0 à 25, choisis de préférence dans le groupe suivant : l'acide laurique, tridécanoïque, myristique, pentadécanoïque, palmitique, margarique, stéarique, nonadécanoïque, arachique, béhénique, lignocérique, cérotique, mélissique, palmitoléique, oléique, érucique, linoléique, linolénique, éléostéarique, arachidonique, le rapport pondéral du composant adoucisseur à l'acide gras étant de préférence de 25:1 à 5:1.

14. Agent de conditionnement selon les revendications 1 à 13, **caractérisé en ce qu'**un parfum, de préférence un parfum hydrophobe, est contenu.

15. Agent de conditionnement selon les revendications 1 à 14, **caractérisé en ce qu'**il existe sous forme de dispersion et possède une taille moyenne de particule inférieure à 500 µm, de manière particulièrement préférée inférieure à 300 µm, de manière tout particulièrement préférée inférieure à 200 µm, en particulier la taille de particule moyenne est comprise entre 0,005 µm et 180 µm.

16. Procédé pour préparer un agent selon l'une quelconque des revendications 1 à 15, **caractérisé en ce que** le composant adoucisseur existe sous forme liquide lors de l'ajout, en particulier à l'état fondu, et est injecté dans le mélange réactionnel.

17. Procédé selon la revendication 16, **caractérisé en ce que** l'ajout du composant adoucisseur et/ou de l'agent cryoprotecteur au mélange réactionnel se réalise par portions.

18. Procédé selon les revendications 16 à 17, **caractérisé en ce que**, l'ajout de l'agent cryoprotecteur se réalise avant l'ajout d'huile de parfum.

19. Procédé selon les revendications 16 à 18, **caractérisé en ce que** l'ajout du composant adoucisseur de textile et de l'agent cryoprotecteur se réalise sous agitation rapide.

20. Procédé selon les revendications 16 à 19, **caractérisé en ce qu'**il s'agit d'un processus continu.

21. Utilisation de l'agent selon l'une quelconque des revendications 1 à 15 pour le conditionnement de tissus textiles, en particulier dans un cycle post-rinçage dans un procédé de lavage.

22. Utilisation de l'agent selon l'une quelconque des revendications 1 à 15, pour le conditionnement de fibres kératiniques, en particulier en utilisant un vaporisateur.

23. Utilisation d'un agent selon l'une quelconque des revendications 1 à 15, en tant que formulation cosmétique.

24. Produit, contenant un agent selon l'une quelconque des revendications 1 à 15 et un vaporisateur.
